# FASCICULE DE BREVET EUROPEEN

(11) **EP 0 709 382 B1**
(45) Date de publication et mention de la délivrance du brevet: **10.11.1999**
(21) Numéro de dépôt: 95402051.7
(22) Date de dépôt: 11.09.1995
(51) Int. Cl.: C07D 307/93, C07D 409/06, C07D 405/06, A61K 31/34, A61K 7/48, A61K 7/06

(54) **Nouveaux composés dérivés aromatiques du dibenzofuranne, compositions pharmaceutiques et cosmétiques les contenant**
Aromatische Dibenzofuranderivate, sie enthaltende pharmazeutische und kosmetische Zusammenstellungen
Aromatic dibenzofurane derivatives, pharmaceutical and cosmetic compositions containing them

(30) Priorité: 28.10.1994 FR 9412989
(43) Date de publication de la demande: 01.05.1996
(73) Titulaire: CENTRE INTERNATIONAL DE RECHERCHES DERMATOLOGIQUES GALDERMA, ( CIRD GALDERMA), 06560 Valbonne (FR)
(72) Inventeur: Charpentier, Bruno, F-06410 Biot (FR); Bernard, Bruno, F-92200 Neuilly sur Seine (FR)
(74) Mandataire: Tezier Herman, Béatrice

(56) Documents cités:
- EP-A- 0 360 637
- GB-A- 2 187 455

## Description

L'invention concerne, à titre de produits industriels nouveaux et utiles, des composés aromatiques polycycliques. Elle concerne également l'utilisation de ces nouveaux composés dans des compositions pharmaceutiques destinées à un usage en médecine humaine ou vétérinaire, ou bien encore dans des compositions cosmétiques.

Dans les documents EP-A-0360637 et GB-A-2187455 il a été décrit des composés benzofuranniques qui diffèrent de composés de formule (I) définie ci-après par leur structure chimique, et en particulier par leur noyau 2,3,4,4a-tétrahydro-4a,10,10-triméthyl-1H-3,9bméthano-dibenzofuranne de formule:

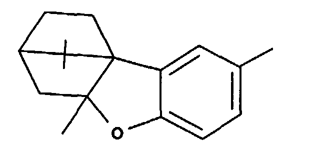

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire, virale et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques, notamment pour l'hygiène corporelle et capillaire.

Les composés selon l'invention, dérivés aromatiques du dibenzofuranne, sont caractérisés par le fait qu'ils présentent la formule générale (I) suivante : 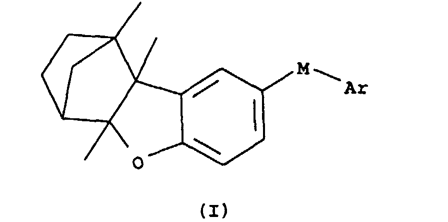 dans laquelle :
* Ar représente l'un des radicaux de formules (II)-(VI) suivantes : 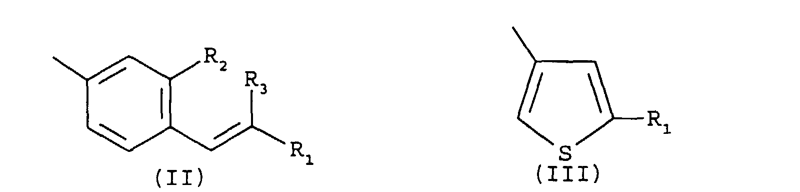 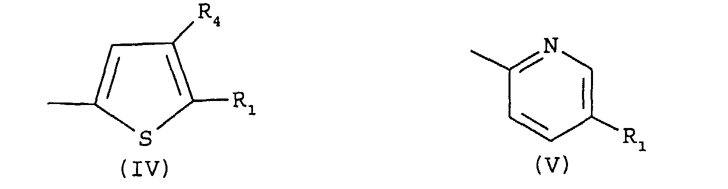 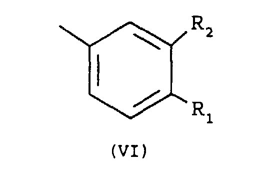
* M représente un radical divalent choisi dans le groupe constitué par les radicaux de formules suivantes (les formules données pouvant être lues de gauche à droite ou inversement) :

   (a) - CR₅R₆ - ,

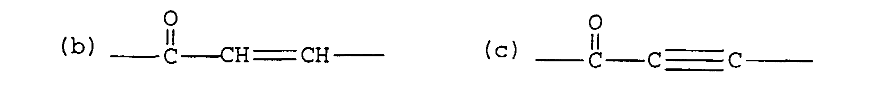

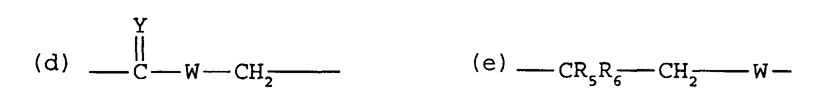

   étant entendu que dans tout ce qui précède :
   - lorsque M représente le radical de formule (a), Ar ne représente pas le radical de formule (VI),
   - R₁ représente :
      (i) un atome d'hydrogène,
      (ii) le radical -CH₃,
      (iii) le radical -(CH₂)ₘ-O-R₈,
      (iv) un radical -OR₈
      (v) un radical 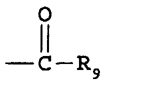
      (vi) un radical -S(O)ₜ R₉,
         m, t, R₈ et R₉ ayant les significations données ci-après,
   - R₂ représente un atome d'hydrogène ou le radical -OR₈,
      R₈ ayant la signification donnée ci-après,
   - R₃ représente un atome d'hydrogène ou un radical alkyle inférieur,
   - R₄ a la même signification que R₁, à la condition que l'un au moins des 2 radicaux R₁ ou R₄ soit l'atome d'hydrogène,
   - soit R₅ et R₆ représentent indépendamment un atome d'hydrogène, un radical alkyle inférieur ou le radical -(X)ₙ - (CH₂)ₚ - R₇,
      soit R₅, R₆ pris ensemble peuvent former un groupe oxo (=O), un groupe thiocetone (=S), oxime ou un groupe (R₁₁-O-N=), epoxy, cyclopropyl, cycloalkyle éventuellement substitué par un atome d'halogène ou un radical alkyle inférieur, ou un groupe dioxolanne (-O-(CH₂)qO-), avec q égal à 2 ou 3,
      X, n , p, R₇ et R₁₁ ayant les significations données ci-après,
   - R₇ représentant un atome d'hydrogène ou un radical -(CO)ᵣ-R₁₀
      r et R₁₀ ayant les significations données ci-après,
   - R₈ représente un atome d'hydrogène, un radical alkyle inférieur, ou un radical acyle inférieur,
   - R₉ représente :
      (i) un atome d'hydrogène,
      (ii) un radical -N (R',R"),
      (iii) un radical -OR₁₁,
         R', R" et R₁₁ ayant les significations données ci-après,
      - R₁₀ représente :
         - un atome d'hydrogène,
         - un radical alkyle,
         - un radical alcenyle,
         - un radical alcynyle,
         - un radical aryle,
         - un radical -OR₁₁,
         - un radical -N (R', R"),
      R', R" et R₁₁ ayant les significations données ci-après,
   - R₁₁ représente un atome d'hydrogène, un radical alkyle ayant de 1 à 20 atomes de carbone, un radical mono ou polyhydroxyalkyle, un radical aryle éventuellement substitué, aralkyle éventuellement substitué, un reste de sucre ou un reste d'aminoacide ou de peptide,
   - R' et R" identiques ou différents représentent un atome d'hydrogène, un radical alkyle inférieur, un radical mono ou polyhydroxyalkyle, un radical aryle éventuellement substitué, un radical aralkyle éventuellement substitué, un reste d'amino acide ou de peptide ou de sucre ou encore R' et R" pris ensemble forment un hétérocycle,
   - W représente un atome d'oxygène ou de soufre ou le groupe -NR₁₂, R₁₂ ayant la signification donnée ci-après,
   - R₁₂ représente un atome d'hydrogène ou le radical -CH₃,
   - X et Y représentent indépendamment un atome d'oxygène ou un atome de soufre
   - m et p, nombres entiers, varient indépendamment de 0 à 10, avec la condition que : lorsque R₇ représente un radical -(CO)ᵣ-R₁₀ et R₁₀ représente le radical -OR₁₁, p ne prend pas la valeur 0.
   - n et r peuvent indépendamment avoir la valeur 0 ou 1,
   - t est égal à 0,1 ou 2.
et les isomères optiques et géométriques desdits composés de formule (I) ainsi que leurs sels dans le cas où R₁, R₄ ou R₇ représente une fonction acide.

Lorsque les composés selon l'invention se présentent sous forme de sels par addition d'une base, il s'agit de préférence de sels d'un métal alcalin ou alcalino-terreux ou encore de zinc ou d'une amine organique.

Lorsque les composés se présentent sous forme de sels, par addition d'un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique.

Par radical alkyle inférieur, on entend un radical ayant de 1 à 6 atomes de carbone et de préférence les radicaux méthyle, éthyle, isopropyle, butyle, tertiobutyle et hexyle.

Par radical alcényle, on entend un radical ayant de 1 à 20 atomes de carbones, linéaire ou ramifié présentant une double liaison.

Par radical alcynyle, on entend un radical ayant de 1 à 20 atomes de carbone, linéaire ou ramifié présentant une triple liaison.

Par radical acyle inférieur, on entend un radical ayant de 1 à 6 atomes de carbone et de préférence les radicaux acétyle, propionyle, pivaloyle.

Les radicaux alkyle ayant de 1 à 20 atomes de carbone sont linéaires ou ramifiés, éventuellement substitués par 1 ou plusieurs atomes de fluor.

Par radical monohydroxyalkyle, on entend un radical ayant de 1 à 6 atomes de carbone, notamment un radical 2-hydroxyéthyle, 2-hydroxypropyle ou 3-hydroxypropyle.

Par radical polyhydroxyalkyle, on entend un radical contenant de 2 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle, on choisit plus particulièrement un radical phényle éventuellement substitué par un ou plusieurs atomes d'halogène, une fonction hydroxyle, nitro ou un groupe méthoxy.

Par radical aralkyle éventuellement substitué, on entend le radical benzyle ou phénéthyle, éventuellement substitué par un ou plusieurs atomes d'halogène, une fonction hydroxyle ou nitro, ou un groupe méthoxy.

Par reste d'amino-acide, on entend un reste dérivant par exemple de l'un des 20 aminoacides de configuration L ou D constitutifs de protéines de mammifères.

Par reste de peptide, on entend un peptide linéaire de 2 à 10 acides aminés.

Par reste de sucre, on entend un reste dérivant par exemple du glucose, du galactose, du mannose ou de l'acide glucuronique.

Les hétérocycles préférés correspondent au radical pipéridino, morpholino, pyrrolidino ou pipérazino, éventuellement substitué en position 4 par un radical alkyle en C₁-C₆ ou mono ou polyhydroxyalkyle tels que définis ci-dessus.

Parmi les composés de formule (I) ci-dessus, on préfère les composés répondant à la formule (I) dans laquelle M représente le radical de formule (e) avec W représentant un atome d'oxygène et Ar le radical de formule (VI), ainsi on peut les représenter suivant la formule (Ia): 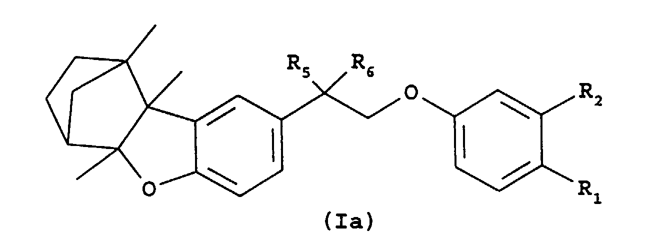 R₁, R₂, R₅ et R₆ ayant les significations données précédemment.

Parmi les composés de formule générale (I), on peut notamment citer les suivants :
4-[3-(1,2,3,4-Tétrahydro-1,4a,9b-triméthyl-1,4-méthanodibenzofuranne-8-yl)-3-oxo-1-(E)-propényl] benzoate de méthyle
Acide 4-[3-(1,2,3,4-Tétrahydro-1,4a,9b-triméthyl-1,4-méthanodibenzofuranne-8-yl)-3-oxo-1-(E)-propényl] benzoïque
4-[3-(1 ,2, 3,4-Tétrahydro-1,4a,9b-triméthyl-1,4-méthanodibenzofuranne-8-yl)-3-oxo-1-(E)-propynyl] benzoate de méthyle
Acide 4-[3-(1,2,3,4-Tétrahydro-1,4a,9b-triméthyl-1,4-méthanodibenzofuranne-8-yl)-3-oxo-1-(E)-propynyl] benzoïque
4-[2-(1,2,3,4-Tétrahydro-1,4a,9b-triméthyl-1,4-méthanodibenzofuranne-8-yl)-2-oxoéthoxy] benzoate de benzyle
Acide 4-[2-(1,2,3,4-Tétrahydro-1,4a,9b-triméthyl-1,4-méthanodibenzofuranne-8-yl)-2-oxoéthoxy] benzoïque
Acide 4-[2-(1,2,3,4-Tétrahydro-1,4a,9b-triméthyl-1,4-méthanodibenzofuranne-8-yl)-2-hydroxyéthoxy] benzoïque
4-[2-(1,2,3,4-Tétrahydro-1,4a,9b-triméthyl-1,4-méthanodibenzofuranne-8-yl)-2-oxoéthoxy]-2-hydroxy benzoate de benzyle
Acide 4-[2-(1,2,3,4-Tétrahydro-1,4a,9b-triméthyl-1,4-méthanodibenzofuranne-8-yl)2-oxoéthoxy]-2-hydroxybenzoïque
Acide 4-[2-(1,2,3,4-Tétrahydro-1,4a,9b-triméthyl-1,4-méthanodibenzofuranne-8-yl)-2-hydroxyéthoxy]-2-hydroxybenzoïque
Acide 4-[2-(1,2,3,4-Tétrahydro-1,4a,9b-triméthyl-1,4-méthanodibenzofuranne-8-yl) -éthoxy]-2-hydroxybenzoïque
4-[(1,2,3,4-Tétrahydro-1,4a,9b-triméthyl-1,4-méthanodibenzofuranne-8-yl)-carbonyl] cinnamate d'éthyle
Acide 4-[(1,2,3,4-Tétrahydro-1,4a,9b-triméthyl-1,4-méthanodibenzofuranne-8-yl)-carbonyl] cinnamique
2-[3-(1,2,3,4-Tétrahydro-1,4a,9b-triméthyl-1,4-méthanodibenzofuranne-8-yl)-3-oxo-1-(E)-propényl]-4 thiophènecarboxylate d'éthyle
Acide 2-[3-(1,2,3,4-Tétrahydro-1,4a,9b-triméthyl-1,4-méthanodibenzofuranne-8-yl)-3-oxo-1-(E)-propényl]-4 thiophènecarboxylique
Acide-4-[3-(1,2,3,4-Tetrahydro-1,4a,9b-triméthyl-1,4-methanodibenzofuranne-8-yl)-3-oxo-(E)-1-propényl]-benzoïque

La présente invention a également pour objet les procédés de préparation des composés de formule (I) qui sont les suivants :

Dans un premier temps, nous synthétisons le noyau :
1,2,3,4-Tétrahydro-1,4a,9b-triméthyl-1,4-méthanodibenzofuranne (THTMDBF).

Ce noyau est obtenu en 2 étapes :
par addition du o-anisyllithium sur la Fenchone, suivie de l'action du pentachlorure de phosphore, du tribromure de phosphore ou d'un acide de lewis. La Fenchone peut être dextrogyre ou lévogyre.

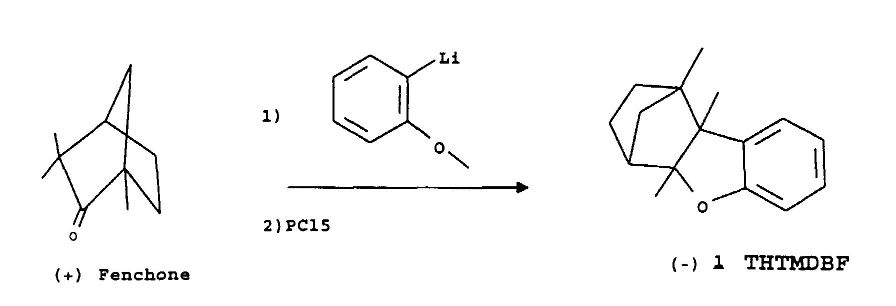

Ce noyau 1,2,3,4-Tétrahydro-1,4a,9b-triméthyl-1,4-méthanodibenzofuranne est utilisé dans la préparation des exemples illustrant les diverses significations indifféremment, soit sous sa forme dextrogyre, soit lévogyre.

### 1) Les composés de formule (I) dans laquelle M a la signification (a) peuvent être préparés comme indiqué ci-dessous :

- soit par réaction de Friedel et Crafts selon le schéma : 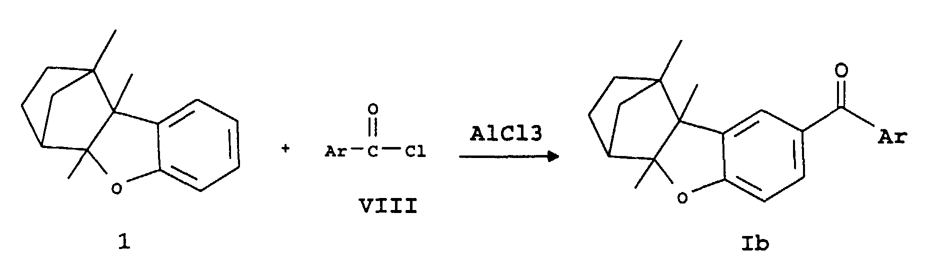 Ar représentant les formules (Il) à (V) indiquées précédemment.
- soit par réaction d'un dérivé organométallique du THTMDBF, tel qu'un organozincique ou un organostannane sur un composé de formule (VIII) selon le schéma ci-après, 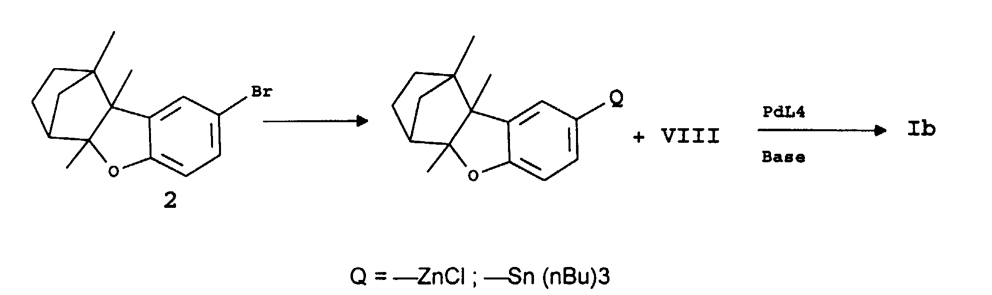
- soit dans des conditions de carbonylation comme indiqué ci-après, 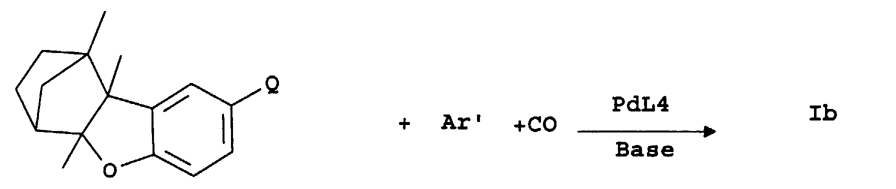 Ar' ayant la signification indiquée ci-dessous:

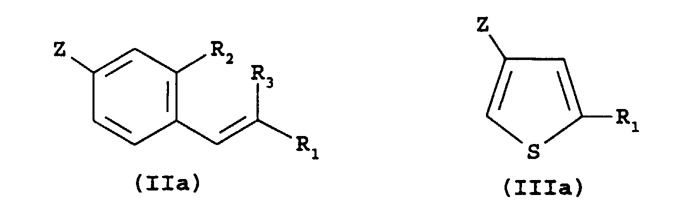

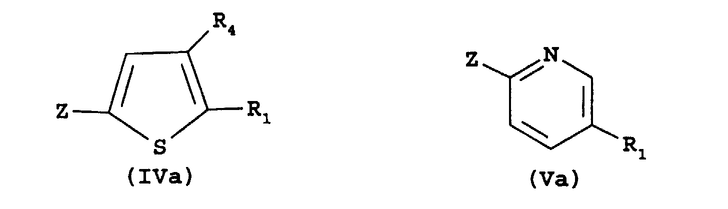

Dans les formules (IIa), (IIIa), (IVa) et (Va), R₁, R₂, R₃, R₄ ont les mêmes significations que dans la définition de la formule générale (I), Z représentant Br, I ou O-SO₂-CF₃

Au cours de ces réactions conduisant aux composés de formule générale (Ib), la fonction R₁ sera éventuellement sous une forme protégée pour être compatible avec les conditions opératoires. Les groupements protecteurs employés sont ceux décrits dans le livre "Protecting groups in organic synthesis" by T.W. Greene, Ed. by John Wiley and Sons (1981).

Le dérivé Ib peut ensuite servir de produit de départ pour la fabrication d'autres composés. Ces dérivés seront obtenus selon les méthodes classiques de synthèse employées en chimie organique telles que celles décrites dans le "Advanced Organic Chemistry" de J. March; John Wiley and Sons, 1985.

Par exemple, on peut procéder aux modifications fonctionnelles des groupes R₁ ou R₄ comme indiqué ci-dessous.

| | |
|---|---|
| acide carboxylique | → ester |
| ester | → acide carboxylique |
| acide | → chlorure d'acide |
| chlorure d'acide | → amide |
| acide | → amide |
| acide | → alcool |
| alcool | → aldéhyde |
| amide | → amine |
| thiol | → thioéther |
| thioéther | → sulfoxyde |
| thioéther | → sulfone |
| acide sulfonique | → ester sulfonique |
| acide sulfonique | → sulfonamide |
| acide sulfinique | → ester sulfinique |

Les composés de formule (I), dans laquelle M représente le cas (a), Ar ayant la signification Il peuvent être obtenus par la séquence suivante : la réduction du cétoacide (IX) en présence d'hydrure de lithium aluminium conduit au diol (X) qui est ensuite oxydé en cétocarboxaldéhyde (XI) par du Pyridiniumchlorochromate. Ce dernier est soumis à une réaction de Horner-Hemmons pour donner les composés de formule Ic.

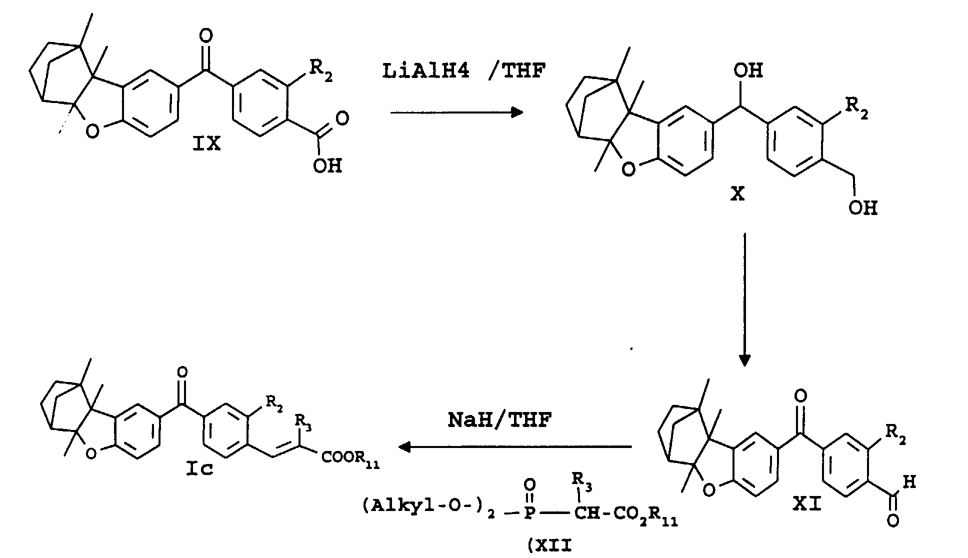

### 2) Les composés de formule (I) dans laquelle M correspond à (b) peuvent être obtenus par une réaction d'aldolisation comme indiqué ci-dessous :

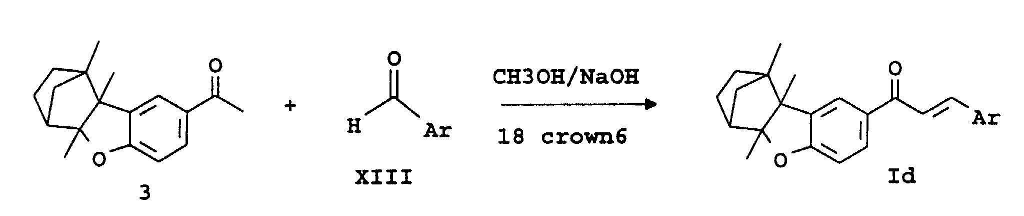

Dans la formule (XIII), Ar représente les radicaux de formules (II) à (VI) définis ci-dessus.
ou si (b) est lu à l'envers :

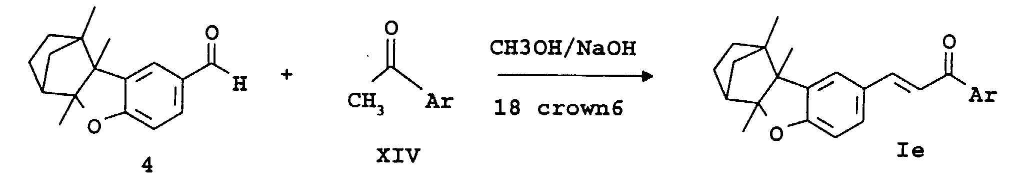

Dans la formule (XIV), Ar représente les radicaux de formules (Il) à (VI) définis ci-dessus.

### 3) Les composés de formule générale (I), dans lesquels M a la signification (c) peuvent être préparés par condensation du dérivé trimethysylilarylacetylène sur le chlorure d'acide fourni à partir du dérivé 5:

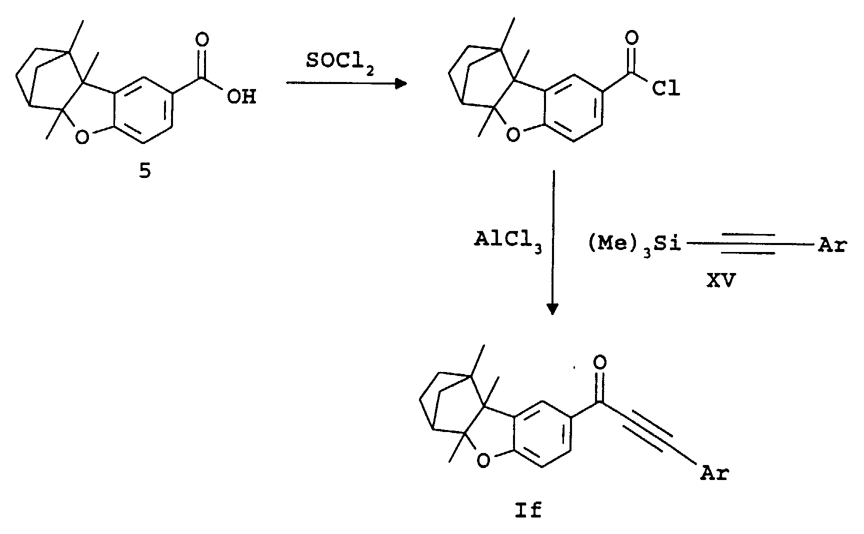

Dans la formule (XV), Ar représente les radicaux de formules (II) à (VI) définis ci-dessus.

Dans le cas où (c) aura pour signification l'enchaînement inverse, la synthèse s'effectuera comme ci-dessous par action du THTMDBFacetylène dans des conditions de carbonylation catalysées par un métal de transition.

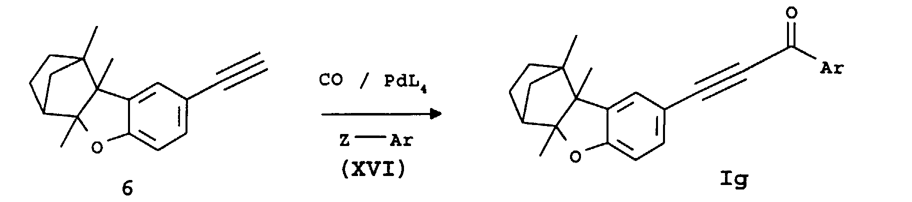

Dans la formule (XVI), Z a la même signification que précédemment et Ar représente les radicaux de formules (Il) à (VI) définis ci-dessus.

### 4) Les composés de formule générale I dans lesquels M a la signification (d) peuvent être préparés de la façon suivante :

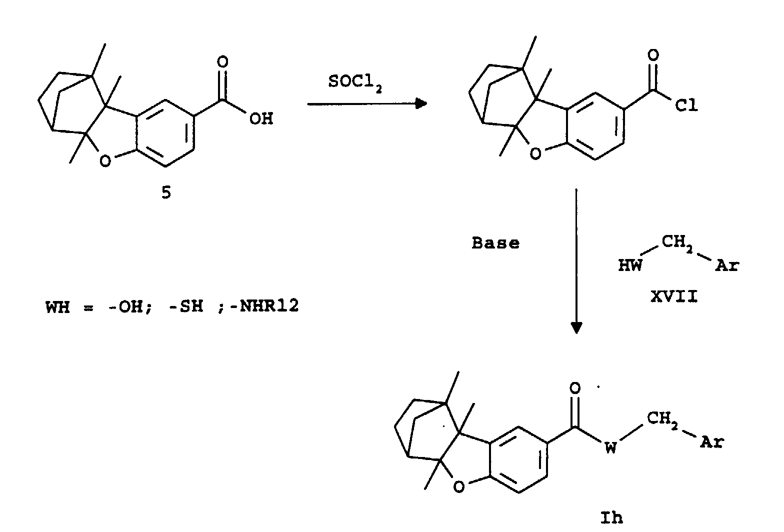

ou selon la séquence, lorsque (d) a la signification de l'enchaînement inverse :

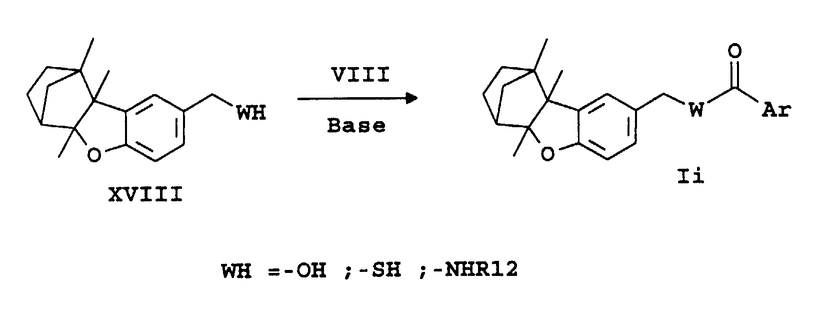

Dans la formule VIII, Ar représente les radicaux de formules (Il) à (VI) définies ci-dessus.

Dans le cas des synthèses des composés de formule (I) dans laquelle M a la signification (d), les substituants R₁, R₂ et R₄ éventuellement présents dans le radical Ar sont, de préférence, convenablement protégés pour être compatibles avec les conditions de couplage.

Cette étape consiste à faire réagir en milieu anhydre, dans un solvant organique tel que le tétrahydrofuranne ou le chlorure de méthylène contenant une base tertiaire (pyridine ou triéthylamine) ou un hydrure alcalin (hydrure de sodium), une forme activée de l'acide TTMDBF carboxylique, telle qu'un chlorure d'acide ou un anhydre mixte sur un composé aromatique porteur d'une fonction hydroxyméthyle, mercaptométhyle ou tertbutoxycarbonylaminométhyle.

Dans le cas où M a la signification (d) avec Y = S et W = NR₁₂, la synthèse de ce dérivé thioamide s'effectuera en employant le réactif de Lawesson [2,4-bis(4-methyloxyphényl)-1,3-dithia-2,4-diphosphetane-2,4-disulfide].

### 5) Les composés de formule générale (I) dans lesquels M a la signification (e) peuvent être préparés de la façon suivante :

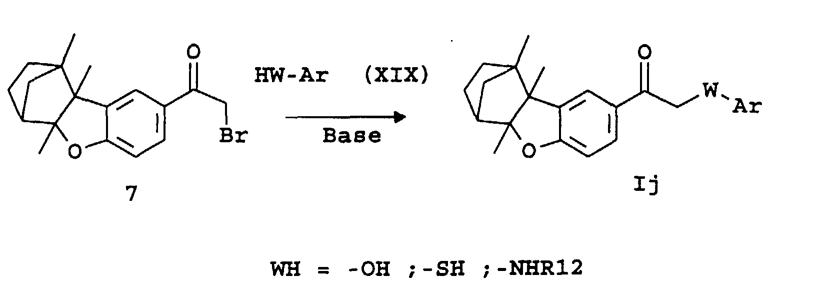

Dans la formule (XIX), Ar représente les radicaux de formules (II) à (VI) définies ci-dessus.
ou selon la séquence, lorsque (d) a la signification de l'enchaînement inverse :

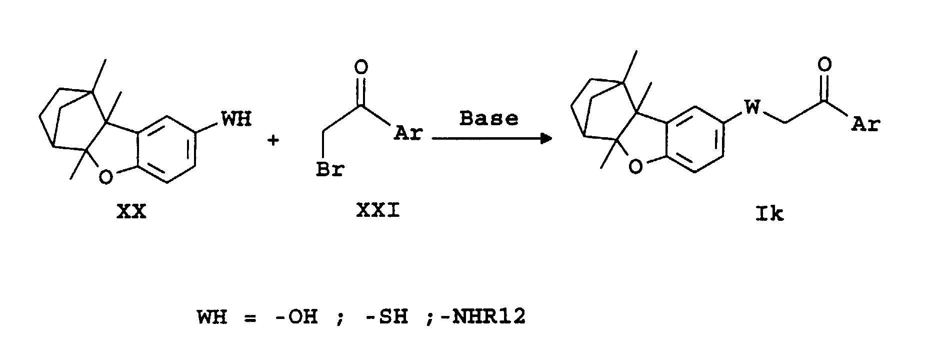

Dans la formule (XXI), Ar représente les radicaux de formules (Il) à (VI) définies ci-dessus.

L'étape principale de cette préparation consiste à faire réagir en milieu anhydre dans un solvant organique tel que le DMF une α-halocétone avec un dérivé aromatique porteur d'une fonction amine, phénol ou thiol, en présence d'une amine tertiaire (pyridine ou triéthylamine) ou d'un hydrure alcalin.

Les groupes R₁, R₂ et R₄ éventuellement présents sur le noyau aromatique Ar sont, de préférence, protégés pour être compatibles avec ces conditions de couplage.

Les dérivés de formule (Ij) et (Ik) peuvent servir de composés de départ pour la préparation d'autres composés.

La présente invention a également pour objet à titre de médicament les composés de formule (I) tels que décrits ci-dessus.

Ces composés présentent une activité agoniste ou antagoniste partielle vis à vis de l'expression d'un ou plusieurs marqueurs biologiques dans le test de différenciation des cellules (F9) de tératocarcinome embryonnaire de la souris (Skin Pharmacol. 3, p.256-267, 1990) et/ou sur la différenciation des kératinocytes humains in vitro (Skin Pharmacol. 3 p.70-85, 1990) en réponse à un traitement par des rétinoïdes. Ces tests sus-mentionnés montrent les activités des composés dans les domaines de la différenciation et de la prolifération. Leurs activités peuvent aussi être mesurées dans des tests de transactivation cellulaire à l'aide de récepteurs recombinants RARs ou RXRs préalablement transfectés. (B.A. Bernard et al., Biochemical and Biophysical Research Communication 1992, vol. 186, 977-983; M.F. Boehm et al. Journal of Medicinal Chemistry, 1994, 37, 408-414).

Les composés selon l'invention conviennent particulièrement bien dans les domaines de traitement suivants :
1) Pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle.
2) Pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen cutané ou muqueux (buccal).
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation.
4) Pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires.
5) Pour traiter d'autres désordres dermatologiques tels que les dermatoses bulleuses et les maladies du collagène.
6) Pour traiter certains troubles ophtalmologiques, notamment les cornéopathies.
7) Pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique.
8) Pour prévenir ou guérir les stigmates de l'atrophie épidermique et/ou dermique induite par les corticostéroïdes locaux ou systémiques, ou tout autre forme d'atrophie cutanée.
9) Pour prévenir ou traiter les troubles de la cicatrisation ou pour prévenir ou réparer les vergetures.
10) Pour lutter contre les troubles de la fonction sébacée tels que l'hyperséborrhée de l'acné ou la séborrhée simple.
11) Dans le traitement ou la prévention des états cancéreux ou précancéreux.
12) Dans le traitement d'affections inflammatoires telles que l'arthrite.
13) Dans le traitement de toute affection d'origine virale au niveau cutané ou général.
14) Dans la prévention ou le traitement de l'alopécie.
15) Dans le traitement d'affections dermatologiques ou générales à composante immunologique.
16) Dans le traitement d'affections du système cardiovasculaire telles que l'artériosclérose.
17) Dans le traitement d'affections respiratoires.

Dans les domaines thérapeutiques mentionnés ci-dessus, les composés selon l'invention peuvent être avantageusement employés en combinaison avec d'autres rétinoïdes, avec les vitamines D ou leurs dérivés, avec des corticostéroïdes, en association avec des anti-radicaux libres, avec des α-hydroxy ou α-céto acides ou leurs dérivés, ou encore avec des bloqueurs de canaux ioniques.

Parmi les vitamines D ou leurs dérivés, on peut citer par exemple les dérivés de la vitamine D₂ ou D₃ et en particulier la 1,25-dihydroxyvitamine D₃.

Parmi les antiradicaux libres, on peut citer par exemple l'α-tocophérol, la Super Oxyde Dismutase, l'Ubiquinol ou certains chélatants de métaux.

Parmi les α-hydroxy ou α-céto acides ou leurs dérivés, on peut citer par exemple les acides lactique, malique, citrique, glycolique, mandélique, tartrique, glycérique ou ascorbique ou dérivés de l'acide salicylique ou leurs sels, amides ou esters.

Parmi les bloqueurs de canaux ioniques, on peut citer par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc aussi pour objet une nouvelle composition médicamenteuse destinée notamment au traitement des affections susmentionées, caractérisée par le fait qu'elle comporte, dans un support pharmaceutiquement acceptable au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection.

Les composés selon l'invention sont généralement administrés à une dose journalière d'environ 0,01 mg/kg à 100 mg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie oculaire, ce sont principalement des collyres.

Ces compositions pour la voie topique ou oculaire contiennent au moins un composé de formule (I) telle que définie ci-dessus, l'un de ses isomères optiques ou géométriques ou un de ses sels, à une concentration de préférence comprise entre 0,001 et 5 % par rapport au poids total de la composition.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Dans le domaine cosmétique, les composés selon l'invention peuvent être avantageusement employés en combinaison avec d'autres rétinoïdes, avec les vitamines D ou leurs dérivés, avec des corticostéroïdes, en association avec des anti-radicaux libres, avec des α-hydroxy ou α-céto acides ou leurs dérivés, ou encore avec des bloqueurs de canaux ioniques.

Les différents produits pris en association avec les composés de la présente invention étant tels que définis ci-dessus.

La présente invention vise donc également une composition cosmétique contenant, dans un support cosmétiquement acceptable, au moins un composé de formule (I) l'un de ses isomères optiques ou géométrique ou l'un de ses sels, cette composition se présentant notamment sous forme d'une crème, d'un lait, d'une lotion, d'un gel, de microsphères ou nanosphères ou vésicules lipidiques ou polymériques, d'un savon ou d'un shampooing.

La concentration en composé de formule (I), dans les compositions cosmétiques est comprise entre 0,001 et 3 % en poids.

Les compositions médicamenteuses et cosmétiques selon l'invention peuvent, en outre, contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféïque ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphényl-imidazolidine 2,4-dione) ; des agents anti-inflammatoires non stéroïdiens; des caroténoïdes et, notamment le b-carotène; des agents anti-psoriatiques tels que l'anthraline et ses dérivés et enfin, les acides eicosa-5,8,11,14- tétraynoïque et eicosa-5,8,11-trynoïque, leurs esters et les amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des anti-oxydants, tels que l'a-tocophérol, le butylhydroxyanisole ou le butylhydroxytoluène.

On va maintenant donner, à titre d'illustration et sans aucun caractère limitatif, plusieurs exemples de préparation des composés actifs de formule (I) selon l'invention ainsi que des exemples de compositions les contenant.

### A. EXEMPLES DE COMPOSES

Les exemples 1 à 7 correspondent à des intermédiaires de synthèse.

Les exemples 8 à 28 correspondent à des composés de formule générale (I).

Tous les produits dont les synthèses sont exposées ci-après ont été caractérisés par RMN du proton (250 MHz) et spectrométrie masse.

### Exemple 1:

### 1,2,3,4-Tetrahydro-1,4a,9b-triméthyl-1,4-methanodibenzofuranne; (THTMDBF).

50 g (0,267 mmoles) de bromoanisole en solution dans 120 ml d'ether éthylique sont traités à 0°C par 200 ml de n-Butylithium (1,6 M dans l'hexane), puis le milieu est laissé sous agitation à température ambiante durant la nuit. On ajoute alors goutte à goutte 41,57 g (273 mmoles) de (+) Fenchone (Fluka) dans 100 ml d'éther éthylique et laisse sous agitation 6 h à température ambiante. Le milieu réactionnel est versé dans 200 ml d'une solution saturée de chlorure d'ammonium.
Après extraction par 600 ml d'éther éthylique, rinçage à l'eau, séchage sur sulfate de magnésium, filtration et évaporation, le résidu est chromatographié sur silice pour conduire à 61,26 g (88%) du 2-0-Anisyl-2-endo-fenchyl Alcool fondant à 62-64°C ; αD = + 78° (c = 1, éthanol).

A une solution de 55,24 g (0.21 mmoles) de 2-0-Anisyl-2-endo-fenchyl Alcool et de 4 g de carbonate de calcium dans 800 ml de chloroforme, on ajoute à -10°C, 57,5 g 0,276 mmoles de pentachlorure de phosphore.

Le mélange réactionnel est agité à température ambiante durant deux heures, puis on ajoute du carbonate de potassium (30 g) et on filtre. Le résidu solide est rincé au chloroforme puis chromatographié sur silice dans un mélange hexane/CH₂Cl₂ (9:1) pour conduire à 31,5 g (65%) du dérivé attendu fondant à 68°C ; αD = - 39,5° (c = 1, éthanol).

La même synthèse effectuée à partir de la (-) fenchone conduit à l'isomère dextrogyre du THTMDBF, fondant à 68°C ; αD = + 36,3° (c = 1, éthanol).

### Exemple 2:

### 8-Bromo-1,2,3,4-Tetrahydro-1,4a,9b-triméthyl-1,4-methanodibenzofuranne

11,42 g (50 mmoles) de (-)1,2,3,4-Tetrahydro-1-4a,9b-triméthyl-1,4-methano dibenzofuranne obtenus à l'exemple 1 sont dissous dans 110 ml de tetrahydrofuranne et sont traités goutte à goutte par une solution contenant 8,9 g de N-Bromosuccinimide (NBS) dans 50 ml de diméthylformamide (DMF). On laisse agiter à température ambiante pendant 2 h 30 puis verse le milieu réactionnel dans de l'eau glacée et extrait par 500 ml d'éther éthylique. Après rincage à l'eau, séchage sur sulfate de magnésium, filtration et évaporation, on isole après chromatographie sur silice dans l'hexane 13,8 g (90%) du dérivé attendu fondant 119,8 °C ; αD = + 4,6° (c = 1, chloroforme).

### Exemple 3:

### 1,2,3,4-Tetrahydro-1,4a,9b-triméthyl-1,4-methanodibenzofuranne-8-yl, methylcétone

A une solution de 14,67 g de chlorure d'aluminium (0,11 mmoles) dans 150 ml de CH₂Cl₂, on ajoute goutte à goute une solution contenant 22,8 g (0.1 mmole) de (-) THTMDBF et 7,8 ml (0,11 mmoles) de chlorure d'acétyle dans 200 ml de dichlorométhane. Le milieu réactionnel est laissé sous agitation pendant 4 h puis est versé dans l'eau glacée et extrait par CH₂Cl₂. Après un traitement habituel de la phase organique, suivi d'une chromatographie sur silice dans le mélange hexane/ether ethylique (85:15), on isole 17,26 g (64%) du dérivé atttendu fondant à 140-142°C ; αD = - 4,3° (c = 1, éthanol).

### Exemple 4:

### 8-(1,2,3,4-Tetrahydro-1,4a,9b-triméthyl-1,4-methanodibenzofuranne)-carboxaldehyde

Dans un tricol sous azote contenant 1,31 g de magnésium, et un cristal d'iode dans 5 ml de tétrahydrofuranne, on ajoute goutte à goutte 13,76 g (44,8 mmoles) de 8-bromo THTMDBF obtenu à l'exemple 2 et laisse le mélange à reflux durant 2 h 30. Après filtration du magnésium, le milieu réactionnel est versé dans l'eau glacée, acidifié à pH1 par de l'acide chlorydrique concentré et extrait par de l'éther. La phase organique est lavée à l'eau, séchée sur sulfate de magnésium, filtrée et évaporée pour conduire à 8,47 g (74%) du dérivé attendu fondant à 144,7°C ; αD = - 7° ( c = 1, chloroforme).

### Exemple 5:

### Acide 8-(1,2,3,4-Tetrahydro-1,4a,9b-triméthyl-1,4-methanodibenzofuranne) carboxylique

Dans un tricol de 250 ml sous azote, on place 622 mg de magnésium, un cristal d'iode et ajoute goutte à goutte en chauffant, une solution contenant 6,55 g (21 mmoles) de 8-bromo-THTMDBF obtenu à l'exemple 2 dans 70 ml de tetrahydrofuranne et laisse à reflux durant 2 heures.
Le milieu reactionnel est ensuite refroidi à -70 °C, saturé en gaz carbonique et laissé revenir à température ambiante durant la nuit.
Après un traitement le milieu réactionnel est versé dans l'eau glacée, acidifié à pH1 par de l'acide chlorydrique concentré et extrait par de l'éther. Après traitement de la phase organique, le résidu est recristallisé dans l'hexane, pour conduire à 4,30 g (75%) du dérivé attendu, fondant à 290-292°C ; αD = - 24,4° (c = 1, diméthylformamide).

### Exemple 6:

### 8-Ethynyl-1,2,3,4-Tetrahydro-1,4a,9b-triméthyl-1,4-methanodibenzofuranne

A une solution de 6,90 ml de diisopropylamine dans 70 ml de tetrahydrofuranne sec, sous argon, on ajoute goutte à goutte à -78°C, 30,5 ml de n-butyl lithium (1,6M dans l'hexane). Cette solution est laissée une heure à -78°C sous agitation, puis on y ajoute goutte à goutte 12 g (44 mmoles) de la methyl cetone obtenue à l'exemple 3 en solution dans 120 ml de tetrahydrofuranne et laisse agiter une heure à -78°C. Le milieu réactionnel est alors traité par 7,1 ml de diethylchlorophosphate puis est laissé revenir à température ambiante à laquelle il est agité 4 heures.
Ce milieu réactionnel est- alors transféré dans une solution de diisopropylamide (97,6 mmoles) à -78°C puis est agité 15 heures à température ambiante. Le milieu réactionnel est alors versé dans de l'eau glacée puis acidifié par de l'acide chlorhydrique 3N. Après extraction par de l'ether ethylique, suivi d'un traitement habituel de la phase organique, le résidu est chromatographié sur silice dans le mélange hexane CH₂Cl₂ (95:15) pour conduire à 4,9 g (44%) du dérivé attendu fondant à 155°C ; αD = - 4,2° (c = 1, chloroforme).

### Exemple 7:

### 1,2,3,4-Tetrahydro-1,4a,9b-triméthyl-1,4-methanodibenzofuranne-8-yl, bromométhylcétone

18 g (66 mmoles) de la cétone obtenue à l'exemple 3 dans 100 ml de dioxanne et 100 ml d'éther sont traités par addition goutte à goutte d'une solution de 3,4 ml de brome dans 35 ml de CH₂Cl₂. On laisse sous agitation le milieu réactionnel 2 heures à température ambiante puis verse dans l'eau glacée et extrait par 800 ml d'éther éthylique. Après séchage et évaporation, le résidu est chromatographié sur silice dans le mélange Hexane/CH₂Cl₂ (50:50). On obtient 19,75 g (80%) du dérivé attendu sous forme d'huile orangée.

### Exemple 8:

### 4-[3-(1,2,3,4-Tetrahydro-1,4a,9b-triméthyl-1,4-methanodibenzofuranne-8-yl)-3oxo-1(E)-propenyl]benzoate de méthyle

Une solution contenant 4 g (14,8 mmoles) (+) THTMDBF-8-yl méthylcétone préparé comme indiqué à l'exemple 3 et 2,42 g (14,7 g) de paraméthoxycarbonylcarboxaldéhyde dans 15 ml de méthanol sont traités par 500 mg de soude et 30 mg d'éther couronne (18 crown-6). Le milieu réactionnel est agité à température ambiante pendant 2 heures puis est versé dans de l'eau acidulée. Après extraction par 200 ml d'éther éthylique suivi du traitement classique et d'une chromatographie sur silice dans le mélange hexane/ether (80:20), on isole 2,28 g (37%) du dérivé attendu fondant à 124-126°C ; αD = + 14,2° (c = 0,54, éthanol).

### Exemple 9:

### Acide-4-[3-(1,2,3,4-Tetrahydro-1,4a,9b-triméthyl-1,4-methanodibenzofuranne-8-yl)-3-oxo-(E)-1-propényl]-benzoïque.

2,2 g (5,28 mmoles) de l'ester méthylique obtenu à l'exemple 8 dans 20 ml de méthanol sont traités par 1 g de soude. Le milieu réactionnel est laissé sous agitation 24 heures à température ambiante.
Le milieu réactionnel est versé dans l'eau glacée, acidifié jusqu'à pH 1 par de l'acide chlorydrique concentré et extrait par de l'acétate d'éthyle. La phase organique est lavée à l'eau, séchée sur sulfate de magnésium et évaporée pour conduire après chromoatographie sur silice dans l'éluant CH₂Cl₂/Méthanol (95:5), on isole 1,15 g (54%) du dérivé attendu de point de fusion 225-227 °C ; αD = + 20,3° (c = 1, éthanol).

### Exemple 10:

### 4-[3-1,2,3,4-Tetrahydro-1,4a,9b-triméthyl-1,4-methanodibenzofuranne-8-yl)-3-oxopropynyl] benzoate de méthyle

1,48 g (4,85 mmoles) de chlorure de l'acide 8-1,2,3,4-Tetrahydro-1,4a,9b-triméthyl-1,4-methanodibenzofuranne-carboxylique obtenu à l'exemple 5 dans 20 ml de CH₂Cl₂ sont traités goutte à goutte à 0°C par une solution contenant 1,12 g (4,_(mmoles) de 4-[triméthylsilylethynyl] benzoate de méthyle et 2,26 g (17 mmoles) de chlorure d'aluminium. On laisse le mélange réactionnel revenir à température ambiante sous agitation en une nuit. Le mélange réactionnel est alors versé dans de l'eau glacée acidulée puis extrait par 350 ml d'éther éthylique.
Après un traitement habituel suivi d'une chromatographie sur silice dans l'éluant CH₂Cl₂/Hexane (80:20), on isole 667 mg (33%) du dérivé attendu.

### Exemple 11:

### Acide-4-[3-(1,2,3,4-Tetrahydro-1,4a,9b-triméthyl-1,4-methanodibenzo furanne-8-yl))-3-oxopropynyl]-benzoïque.

0,66 g de l'ester méthylique obtenu à l'exemple 10, en solution dans 10 ml de tetrahydrofuranne sont traités par 200 mg d'hydroxyde de lithium monohydraté.
Le mélange réactionnel est chauffé à reflux pendant 6 h. Après le même traitement qu'à l'exemple 5, suivi d'une recristallisation dans le cyclohexane, on isole 200 mg du dérivé attendu de point de fusion 165-170°C.

### Exemple 12:

### Acide 4-[2-(1,2,3,4-Tetrahydro-1,4a,9b-triméthyl-1,4-methanodibenzo furanne-8-yl)-2-oxoéthoxy]-benzoique

### Exemple 12a:

### 4-[2-(1,2,3,4-Tetrahydro-1,4a,9b-triméthyl-1,4-methanodibenzofuranne-8-yl)-2-oxoéthoxy]-benzoate de benzyle

Dans un ballon contenant 605 mg d'hydrure de sodium (80% dans l'huile) en suspension dans 50 ml de diméthylformamide, on ajoute goutte à goutte 4,57 g de parahydroxybenzoate de benzyle et laisse agiter à température ambiante 2 heures. On ajoute alors goutte à goutte au milieu réactionnel ainsi constitué 7 g (20 ml de la bromométhylcétone obtenue à l'exemple 6 dans 70 ml de diméthylformamide et laisse agiter à température ambiante durant 4 heures. Après le même traitement que pour l'exemple 2, on isole après chromatographie sur silice dans le gradient d'éluant (hexane /CH₂Cl₂ de 30:70 à 20:80), 8,90 g (90%) du dérivé attendu.

### Exemple 12b:

### Acide 4-[2-(1,2,3,4-Tetrahydro-1,4a,9b-triméthyl-1,4-methanodibenzo furanne-8-yl)-2-oxoéthoxy]-benzoique

4,41 g (8,8 mmoles) de l'ester benzylique obtenu à l'exemple 12a dans 70 ml de dioxanne sont hydrogénés en présence de 132 mg de Palladium (10%) sur charbon à 60°C sous une pression d'hydrogène de 7 bars durant 8 heures. Après filtration sur celite du milieu réactionnel et évaporation, le résidu est chromatographié sur silice dans l'éluant CH₂Cl₂/Ether éthylique (95:5). Après empatage dans l'hexane, on isole 2,63 g (73%) du dérivé attendu fondant à 187,5°C.

### Exemple 13:

### Acide 4-[2-(1,2,3,4-Tetrahydro-1,4a,9b-triméthyl-1,4-methanodibenzo furanne-8-yl)-2-hydroxyéthoxy]-benzoique

4,41 g (8,9 mmoles) de l'ester benzylique obtenu à l'exemple 9a dans 90 ml de dioxanne sont hydrogénés en présence de 660 mg de Palladium (10%) sur charbon sous une pression d'hydrogène de 7 bars à température ambiante durant 3 heures. Après filtration sur célite du milieu réactionnel, évaporation chromatographie sur silice dans l'éluant CH₂Cl₂/Méthanol (9:1), on isole 1,60 g (63%) du dérivé attendu fondant à 123,2°C.

### Exemple 14:

### 4-[2-(1,2,3,4-Tetrahydro-1,4a,9b-triméthyl-1,4-methanodibenzofuranne-8-yl)-2-oxoéthoxy]-2-hydroxybenzoate de benzyle

Dans un ballon contenant 0.6 g d'hydrure de sodium à 80% dans l'huile, en suspension dans 50 ml de diméthylformamide, on ajoute 4,9 g (0,02 mmoles) de 2,4-dihydroxybenzoate de benzyle et laisse agiter durant 2 heures à température ambiante. On ajoute alors goutte à goutte 7 g (0.02 mmoles) de la bromométhylcétone obtenue à l'exemple 7. Le milieu réactionnel est laissé sous agitation 3 heures à température ambiante. Après le même traitement qu'à l'exemple 2, suivi d'une chromatographie sur silice dans l'éluant hexane/CH₂Cl₂ (2:8) et d'une recristallisation dans l'hexane, on isole 7,42 g (63%) du dérivé attendu fondant à 98,4°C.

### Exemple 15:

### Acide 4-[2-(1,2,3,4-Tetrahydro-1,4a,9b-triméthyl-1,4-methanodibenzo furanne-8-yl)-2-oxoéthoxy]-2-hydroxybenzoique

3,7 g (7,2 mmoles) de l'ester benzylique obtenu à l'exemple 14 en solution dans 50 ml de dioxanne contenant 0.1 ml d'acide acétique, sont hydrogénés sous une pression de 7 bars en presence de 110 mg de Palladium (10%) sur charbon pendant 3 heures à 60°C. Après le même traitement que pour l'exemple 14, on isole 2,19 g (72%) du dérivé attendu fondant à 186-188 °C.

### Exemple 16:

### Acide 4-[2-(1,2,3,4-Tetrahydro-1,4a,9b-triméthyl-1,4-methanodibenzo furanne-8-yl)-2-hydroxyethoxy]-2-hydroxybenzoique

3,67 g (7,16 mmoles) de l'ester benzylique obtenu à l'exemple 14 en solution dans 50 ml de dioxanne sont hydrogénés sous une pression de 7 bars d'hydrogène en présence de 550 mg de palladium (10%) sur charbon à température ambiante durant 4 heures Après le même traitement qu'à l'exemple 14 suivi d'une recristallisation dans le mélange ether disopropyliquel/hexane, on isole 1;81 g (60%) du dérivé attendu fondant 168-170 °C.

### Exemple 17:

### Acide 4-[2-(1,2,3,4-Tetrahydro-1,4a,9b-triméthyl-1,4-methanodibenzo furanne-8-yl) éthoxy]-2-hydroxybenzoique

2,05 g (4 mmoles) de l'ester benzylique obtenu à l'exemple 11 en solution dans 30 ml de dioxanne sont hydrogénés sous une pression de 7 bars à 70 °C, pendant 6 heures. On isole après le même traitement qu'à l'exemple 11 suivi d'une chromatographie sur silice dans le CH₂Cl₂, 0.83 g (51%) du dérivé attendu de point de fusion 212-214°C.

### Exemple 18:

### Produits intermédiaires du 4-[(1,2,3,4-Tetrahydro-1,4a,9b-triméthyl-1,4-methanodibenzofuranne-8-yl)carbonyl]cinnamate d'éthyle

### Exemple 18a:

### 4-[(1,2,3,4-Tetrahydro-1,4a,9b-triméthyl-1,4-methanodibenzofuranne-8-yl)carbonyl]benzoate de méthyle

A une solution composée de 6,76 g (29,6 mmoles) de (+) THTMDBF et 5,72 g (28,8 mmoles) de chlorure d'acide du monotéréphtalate de méthyle dans 130 ml de CH₂Cl₂, on ajoute goutte à goutte 5,92 g (44 mmoles) de chlorure d'aluminium et laisse agiter à température ambiante durant la nuit.
Après le même traitement qu' à l'exemple 13a, suivi d'une chromatographie sur silice dans le mélange d'éluant CH₂Cl₂/hexane (60:40), on isole 4,47 g (40%) du dérivé attendu fondant à 150°C.

### Exemple 18b:

### Acide 4-[(1,2,3,4-Tetrahydro-1,4a,9b-triméthyl-1,4-methanodibenzo-furanne-8-yl)-carbonyl]benzoïque

2,52 g (6,44 mmoles) de l'ester obtenu à l'exemple 18a en solution dans 30 ml de méthanol sont traités par 2,5 g de soude et chauffés à reflux pendant 3 heures. Après le même traitement qu'à l'exemple 5, suivi d'une recristallisation dans l'hexane, on isole 2,35 g (97%) du produit attendu fondant à 262-264°C. aD = 22,1° (c = 1, diméthylformamide).

### Exemple 18c:

### 4-[(1,2,3,4-Tetrahydro-1,4a,9b-triméthyl-1,4-methanodibenzofuranne-8-yl)hydroxymethyl]phenylcarbinol

A une suspension de 1,14 g d'hydrure de Lithium aluminium dans 10 ml de tetrahydrofuranne, on ajoute goutte à goutte une solution de 3,76 g (10 mmoles) de l'acide obtenu à l'exemple 18b et laisse agiter 3 heures à température ambiante. On neutralise à 0°C le milieu réactionnel par addition goutte à goutte d'une solution saturée de chlorure d'ammonium. Le précipité est filtré, lavé par de l'hexane et séché pour conduire à 3,13 g (86%) du dérivé attendu fondant 113 - 115 °C.

### Exemple 18d:

### 4-[(1,2,3,4-Tetrahydro-1,4a,9b-triméthyl-1,4-methanodibenzofuranne-8-yl)carbonyl]phenylcarboxaldéhyde

3,1 g (8,5 mmoles) du diol obtenu à l'exemple 18c en solution dans 60 ml de CH₂Cl₂ sont traités par 5,5 g de Pyridinium chlorochromate. La réaction est laissée sous agitation à température ambiante pendant 3 heures. Le milieu réactionnel est alors filtré sur célite. La phase organique est lavée avec une solution saturée de Chlorure d'ammonium, rincée à l'eau, séchée et évaporée pour conduire après chromatographie sur silice dans le mélange d'éluant CH₂Cl₂/hexane (9:1) à 2 g (66%) du dérivé attendu fondant à 138-142°C.

### Exemple 19:

### 4-[(1,2,3,4-Tetrahydro-1,4a,9b-triméthyl-1,4-methanodibenzofuranne-8-yl)carbonyl]cinnamate d'éthyle

1,65 ml (8,3 mmoles) de diéthylphosphonoacétate d'éthyle en solution dans 100 ml de tetrahydrofuranne sont traités par 500 mg de hydrure de sodium (80°C dans l'huile) et laissés sous agitation pendant 2 heures à température ambiante.

A ce milieu reactionnel on ajoute une solution contenant 100 mg d'éther couronne (15 crown-5) et 2 g (5,5 mmmoles) de l'aldéhyde obtenu à l'exemple 18d en solution dans 30 ml de tetrahydrofuranne. Le milieu réactionnel est laissé sous agitation pendant 2 h 30 à température ambiante. Le milieu réactionnel est neutralisé par de l'acide chlorhydrique 1N. La phase organique est lavée, séchée et évaporée. Le résidu est chromatographié sur silice dans le CH₂Cl₂, et après une recristallisation dans l'hexane, on isole 2,18 g (75%) du produit attendu fondant à 115-116°C.

### Exemple 20:

### Acide 4-[(1,2,3,4-Tetrahydro-1,4a,9b-triméthyl-1,4-methanodibenzofuranne-8-yl)carbonyl]cinnamique

1,5 g (3,5 mmoles) de l'ester éthylique obtenu à l'exemple 19 sont saponifiés dans les conditions décrites à l'exemple 9 pour conduire après recristallisation dans mélange éthanol/H2O (4:1) à 1,2 g (85%) de l'acide attendu fondant à 242-243-°C ; αD = + 11,2 (C = 1, chloroforme).

### Exemple 21:

### 2-[3-(1,2,3,4-Tetrahydro-1,4a,9b-triméthyl-1,4-methanodibenzofuranne-8-yl)-3oxo-1-(E)-propényl]4-thiophénecarboxylate d'éthyle

2,70 g (10 mmoles) dans la méthylacétone obtenue à l'exemple 3 sont convertis en chalcone en présence de 1,7 g de 4-Ethoxycarbonyl thiophene carboxaldéhyde dans les conditions décrites à l'exemple 8 pour conduire après recristallisation dans l'acetate d'ethyle à 2,2 g (52%) du dérivé attendu fondant à 149-151°C.

### Exemple 22:

### Acide 2-[3-(1,2,3,4-Tetrahydro-1,4a,9b-triméthyl-1,4-methanodibenzo furanne-8-yl)-3-oxo-1-(E)-propényl]4-thiophènecarboxylique

2,1 g (4,97 mmoles) de l'ester éthylique obtenu à l'exemple 20 sont saponifiés dans les conditions décrites à l'exemple 8 pour conduire après le même traitement et recristallisation dans l'acétate déthyle à 1,5 g (75%) du dérivé attendu fondant à 226-228°C.

### Exemple 23:

### Acide-4-[3-(1,2,3,4-Tetrahydro-1,4a,9b-triméthyl-1,4-methanodibenzofuranne-8-yl)-3-oxo-(E)-1-propényl]-benzoïque

On réalise la même synthèse qu'à l'exemple 9, en partant de 2 g (4,8 mmoles) permet d'obtenir 1 g (49 %) du dérivé levogyre attendu fondant à 227°C; aD= -19,1° ( c=1, éthanol).

### Exemple 24:

Activités biologiques de certains composés exemplifiés ci-dessus

| EXEMPLES | Affinités pour les récepteurs Kd (nM) ^{(a)} | | | Activité antagoniste sur la différenciation des cellules F9 (IC₅₀, nM)^{(b)} |
|---|---|---|---|---|
| | RAR α | RAR β | RAR γ | |
| 12 | 3200 | 745 | 481 | 40 |
| 13 | >3200 | 230 | 40 | 36 |
| 15 | 2500 | 114 | 150 | 55 |
| 17 | >10000 | 315 | 2250 | 270 |
| 23 | 220 | 53 | 27 | 19 |

| | | | | |
|---|---|---|---|---|
| (a) Les affinités pour les RARs sont déterminées dans les conditions décrites dans la publication : B. Martin et al.: Selective Synthetic Ligands for Human Nuclear Retinoic Acid Receptors. Skin Pharmacol. 1992; 5:57-65. | | | | |
| (b)L'activité antagoniste est déterminée en co-incubant un agoniste de référence (N-[4-Carboxybenzyl)-(5,6,7,8-tetrahydro-5,5,8,8-tetramethyl-2-naphtalenyl) amine (F9 AC₅₀ = 4nM) et différentes concentrations du rétinoïde à tester. Ces expériences sont effectuées dans les conditions de détermination de l'activité sur la différenciation de cellules F9 selon la publication ci-dessous : Darmon, M., M. Rocher, M.T. Cavey, B. Martin, T. Rabilloud, C. Delescluse and B. Shroot. Biological activity of retinoids correlates with affinity for nuclear receptors, but not for cytosolic binding protein. Skin Pharmacol. 1:161-175 (1988). | | | | |

### B. EXEMPLES DE FORMULATION

### 1) VOIE ORALE

### Exemple 25:

On prépare la composition suivante sous la forme d'un comprimé de 0,8 g

| | |
|---|---|
| Composé de l'exemple 9 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administre à un individu adulte 1 à 3 comprimés par jour pendant 3 à 6 mois selon la gravité du cas traité.

### Exemple 26:

On prépare une suspension buvable, destinée à être conditionnée en ampoules de 5 ml

| | |
|---|---|
| Composé de l'exemple 15 | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p. | 5 ml |

Pour le traitement de l'acné, on administre à un individu adulte 1 ampoule par jour pendant 3 mois selon la gravité du cas traité.

### Exemple 27:

On prépare la formulation suivante destinée à être conditionnée en gélules :

| | |
|---|---|
| Composé de l'exemple 16 | 0,025 g |
| Amidon de maïs | 0,060 g |
| Lactose q.s.p. | 0,300 g |

Les gélules utilisées sont constituées de gélatine, d'oxyde de titane et d'un conservateur.

Dans le traitement du psoriasis, on administre à un individu adulte, 1 gélule par jour pendant 30 jours.

### 2) VOIE TOPIQUE

### Exemple 28:

On prépare la crème Eau-dans l'Huile non ionique suivante :

| | |
|---|---|
| Composé de l'exemple 8 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p | 100,000 g |

Cette crème est appliquée sur une peau psoriatique 1 à 2 fois par jour pendant 30 jours.

### Exemple 29:

On prépare un gel en réalisant la formulation suivante :

| | |
|---|---|
| Composé de l'exemple 9 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel est appliqué sur une peau atteinte de dermatose ou une peau acnéique 1 à 3 fois par jour pendant 6 à 12 semaines selon la gravité du cas traité.

### Exemple 30:

On prépare une lotion antiséborrhéique en procédant au mélange des ingrédients suivants :

| | |
|---|---|
| Composé de l'exemple 10 | 0,030 g |
| Propylène glycol | 5,000 g |
| Butylhydroxytoluène | 0,100 g |
| Ethanol (à 95°) q.s.p. | 100,000 g |

Cette lotion est appliquée deux fois par jour sur un cuir chevelu séborrhéique et on constate une amélioration significative dans un délai compris entre 2 et 6 semaines.

### Exemple 31:

On prépare une composition cosmétique contre les effets néfastes du soleil en procédant au mélange des ingrédients suivants :

| | |
|---|---|
| Composé de l'exemple 12 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition est appliquée quotidiennement, elle permet de lutter contre le vieillissement photoinduit.

### Exemple 32:

On prépare la crème Huile dans l'Eau non ionique suivante :

| | |
|---|---|
| Composé de l'exemple 13 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée q.s.p. | 100,000 g |

Cette crème est appliquée sur une peau psoriatique 1 à 2 fois par jour pendant 30 Jours.

### Exemple 33:

On prépare un gel topique en procédant au mélange des ingrédients suivants :

| | |
|---|---|
| Composé de l'exemple 15 | 0,050 g |
| Ethanol | 43,000 g |
| a-tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau 9,300 g | |
| Propylène glycol qsp | 100,000 g |

Ce gel est appliqué dans le traitement de l'acné 1 à 3 fois par jour pendant 6 à 12 semaines selon la gravité du cas traité.

### Exemple 34:

On prépare une lotion capillaire anti-chute et pour la repousse des cheveux en procédant au mélange des ingrédients suivants :

| | |
|---|---|
| Composé de l'exemple 16 | 0,05 g |
| Composé vendu sous la dénomination "Minoxidil" | 1,00 g |
| Propylène glycol | 20,00 g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On applique cette lotion 2 fois par jour pendant 3 mois sur un cuir chevelu ayant subi une chute de cheveu importante.

### Exemple 35:

On prépare une crème anti-acnéique en procédant au mélange des ingrédients suivants :

| | |
|---|---|
| Composé de l'exemple 17 | 0,050 g |
| Acide rétinoïque | 0,010 g |
| Mélange de stéarates de glycérol et de polyéthylène glycol (75 moles) vendu sous le nom de "Gelot 64" par la société | |
| "GATTEFOSSE" | 15,000 g |
| Huile de noyau polyoxyéthylénée à 6 moles d'oxyde d'éthylène vendue sous le nom de "Labrafil M2130 CS" par la société | |
| "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000 g |

Cette crème est appliquée sur une peau atteinte de dermatose ou une peau acnéique 1 à 3 fois par jour pendant 6 à 12 semaines.

### Exemple 36:

On prépare une crème huile dans l'eau en réalisant la formulation suivante :

| | |
|---|---|
| Composé de l'exemple 19 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p | 100,000 g |

Cette crème est appliquée 2 fois par jour sur une peau atteinte de dermatose pendant 30 jours.

### Exemple 37:

On prépare la crème de type huile dans l'eau suivante :

| | |
|---|---|
| Acide lactique | 5,000 g |
| Composé de l'exemple 20 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Eau q.s.p | 100,000 g |

Cette crème est appliquée 1 fois par jour, elle aide à lutter contre le vieillissement qu'il soit photoinduit ou chronologique.

## Revendications

1. Composés, caractérisés en ce qu'ils présentent la formule (I) suivante : 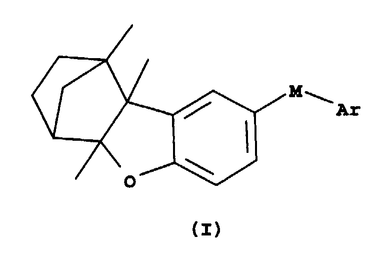
dans laquelle :
* Ar représente l'un des radicaux de formules (II)-(VI) suivantes : 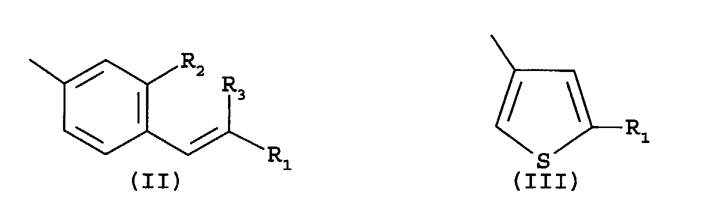 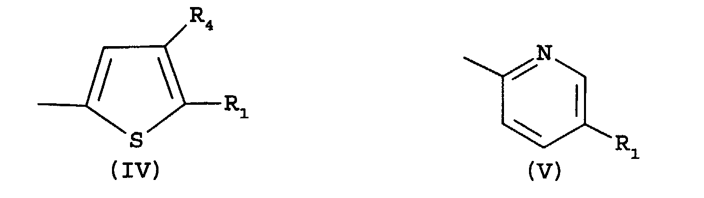 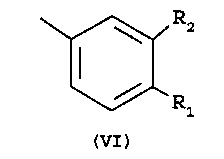
* M représente un radical divalent choisi dans le groupe constitué par les radicaux de formules suivantes (les formules données pouvant être lues de gauche à droite ou inversement) :
(a) - CR₅R₆- , 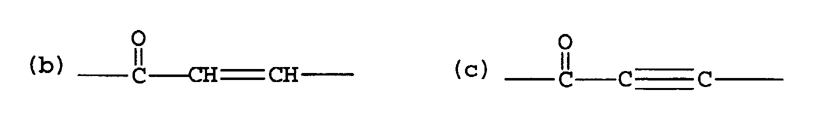 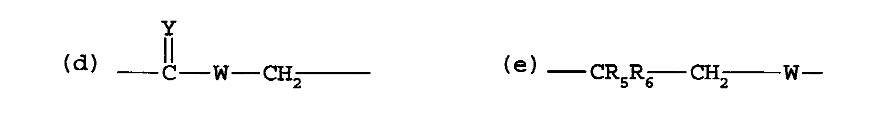
étant entendu que dans tout ce qui précède :
- lorsque M représente le radical de formule (a), Ar ne représente pas le radical de formule (VI),
- R₁ représente :
(i) un atome d'hydrogène,
(ii) le radical -CH₃,
(iii) le radical -(CH₂)ₘ-O-R_{8,}
(iv) un radical -OR₈
(v) un radical 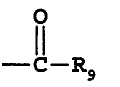
(vi) un radical -S(O)ₜ R_{9 ,}
m, t, R₈ et R₉ ayant les significations données ci-après,
- R₂ représente un atome d'hydrogène ou le radical -OR₈,
R₈ ayant la signification donnée ci-après,
- R₃ représente un atome d'hydrogène ou un radical alkyle ayant de 1 à 6 atomes de carbone,
- R₄ a la même signification que R₁, à la condition que l'un au moins des 2 radicaux R₁ ou R₄ soit l'atome d'hydrogène,
- soit R₅ et R₆ représentent indépendamment un atome d'hydrogène, un radical alkyle ayant de 1 à 6 atomes de carbone ou le radical -(X)ₙ - (CH₂)ₚ - R₇ ,
soit R₅, R₆ pris ensemble peuvent former un groupe oxo (=O), un groupe thiocetone (=S), oxime ou un groupe (R₁₁-O-N=), epoxy, cyclopropyl, cycloalkyle éventuellement substitué par un atome d'halogène ou un radical alkyle ayant de 1 à 6 atomes de carbone, ou un groupe dioxolanne (-O-(CH₂)q-O-), avec q égal à 2 ou 3,
X, n , p, R₇ et R₁₁ ayant les significations données ci-après,
- R₇ représentant un atome d'hydrogène ou un radical -(C0)ᵣ-R₁₀ r et R₁₀ ayant les significations données ci-après,
- R₈ représente un atome d'hydrogène, un radical alkyle ayant de 1 à 6 atomes de carbone, ou un radical acyle ayant de 1 à 6 atomes de carbone,
- R₉ représente :
(i) un atome d'hydrogène,
(ii) un radical -N (R',R"),
(iii) un radical -OR_{11,}
R', R" et R₁₁ ayant les significations données ci-après,
- R₁₀ représente :
- un atome d'hydrogène,
- un radical alkyle,
- un radical alcenyle,
- un radical alcynyle,
- un radical aryle,
- un radical -OR₁₁-,
- un radical -N (R', R"),
R', R" et R₁₁ ayant les significations données ci-après,
- R₁₁ représente un atome d'hydrogène, un radical alkyle ayant de 1 à 20 atomes de carbone, un radical mono ou polyhydroxyalkyle, un radical aryle éventuellement substitué par un ou plusieurs atomes d'halogène, une fonction hydroxyle, nitro ou un groupe méthoxy; un radical aralkyle éventuellement substitué par un ou plusieurs atomes d'halogène, une fonction hydroxyle, nitro ou un groupe méthoxy; ou un reste de sucre,
- R' et R" identiques ou différents représentent un atome d'hydrogène, un radical alkyle ayant de 1 à 6 atomés de carbone, un radical mono ou polyhydroxyalkyle, un radical aryle éventuellement substitué par un ou plusieurs atomes d'halogène, une fonction hydroxyle, nitro ou un groupe méthoxy; un radical aralkyle éventuellement substitué par un ou plusieurs atomes d'halogène, une fonction hydroxyle, nitro ou un groupe méthoxy; un reste d'amino acide ou de peptide ou de sucre ou encore R' et R" pris ensemble forment un hétérocycle,
- W représente un atome d'oxygène ou de soufre ou le groupe -NR₁₂,
R₁₂ ayant la signification donnée ci-après,
- R₁₂ représente un atome d'hydrogène ou le radical -CH₃,
- X et Y représentent indépendamment un atome d'oxygène ou un atome de soufre
- m et p, nombres entiers, varient indépendamment de 0 à 10, avec la condition que :
lorsque R₇ représente un radical -(CO)ᵣ-R₁₀ et R₁₀ représente le radical -OR₁₁, p ne prend pas la valeur 0.
- n et r peuvent indépendamment avoir la valeur 0 ou 1,
- t est égal à 0,1 ou 2.
et les isomères optiques et géométriques desdits composés de formule (1) ainsi que leurs sels dans le cas où R₁, R₄ ou R₇ représente une fonction acide.

2. Composés selon la revendication 1, caractérisés en ce qu'ils se présentent sous forme de sels par addition d'une base ou d'un acide.

3. Composés selon l'une des revendications précédentes, caractérisés par le fait que le radical alkyle ayant de 1 à 6 atomes de carbone est choisi dans le groupe constitué par les radicaux méthyle, éthyle, isopropyle, butyle, tertiobutyle et hexyle.

4. Composés selon l'une des revendications précédentes, caractérisés par le fait que le radical acyle ayant de 1 à 6 atomes de carbone est choisi dans le groupe constitué par les radicaux acétyle, propionyle, pivaloyle.

5. Composés selon l'une des revendications précédentes, caractérisés par le fait que le radical monohydroxyalkyle est choisi dans le groupe constitué par les radicaux 2-hydroxyéthyle, 2-hydroxypropyle ou 3-hydroxypropyle.

6. Composés selon l'une des revendications précédentes, caractérisés par le fait que le radical polyhydroxyalkyle est choisi dans le groupe constitué par les radicaux 2,3-dihydroxypropyle, 2,3,4-trihydroxybutyle, 2,3,4,5-tétrahydroxypentyle ou le reste du pentaérythritol.

7. Composés selon l'une des revendications précédentes, caractérisés par le fait que le radical aryle est un radical phényle éventuellement substitué par un ou plusieurs atomes d'halogène, une fonction hydroxyle, nitro ou un groupe méthoxy.

8. Composés selon l'une des revendications précédentes, caractérisés par le fait que par radical aralkyle est le radical benzyle ou phénéthyle, éventuellement substitué par un ou plusieurs atomes d'halogène, une fonction hydroxyle ou nitro, ou un groupe méthoxy.

9. Composés selon l'une des revendications précédentes, caractérisés par le fait que le reste de sucre est un reste dérivant du glucose, du galactose, du mannose ou de l'acide glucuronique.

10. Composés selon l'une des revendications précédentes, caractérisés par le fait que l'hétérocycle est un radical pipéridino, morpholino, pyrrolidino ou pipérazino, éventuellement substitué en position 4 par un radical alkyle en C₁-C₆ ou mono ou polyhydroxyalkyle.

11. Composés selon l'une quelconque des revendications précédentes, caractérisés par le fait qu'ils présentent la formule (la) suivante :

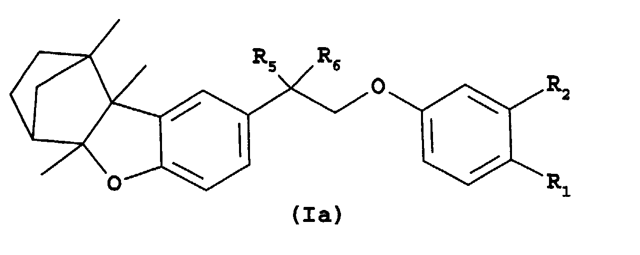

12. Composés selon l'une quelconque des revendications 1 à 10, caractérisés par la fait que'ils sont choisis dans le groupe constitué par:
4-[3-(1,2,3,4-Tétrahydro-1,4a,9b-triméthyl-1,4-méthanodibenzofuranne-8-yl)-3-oxo-1(E)-propényl] benzoate de méthyle
Acide 4-[3-(1,2,3,4-Tétrahydro-1,4a,9b-triméthyl-1,4-méthanodibenzofuranne-8-yl)3-oxo-1-(E)-propényl] benzoïque
4-[3-(1,2,3,4-Tétrahydro-1,4a,9b-triméthyl-1,4-méthanodibenzofuranne-8-yl)-3-oxo-1-(E)-propynyl] benzoate de méthyle
Acide 4-[3-(1,2,3,4-Tétrahydro-1,4a,9b-triméthyl-1,4-méthanodibenzofuranne-8-yl)-3-oxo-1-(E)-propynyl] benzoïque
4-[2-(1,2,3,4-Tétrahydro-1,4a,9b-triméthyl-1,4-méthanodibenzofuranne-8-yl)-2-oxoéthoxy] benzoate de benzyle
Acide 4-[2-(1,2,3,4-Tétrahydro-1,4a,9b-triméthyl-1,4-méthanodibenzofuranne-8-yl)-2-oxoéthoxy] benzoïque
Acide 4-[2-(1,2,3,4-Tétrahydro-1,4a,9b-triméthyl-1,4-méthanodibenzofuranne-8-yl)-2-hydroxyéthoxy] benzoïque
4-[2-(1,2,3,4-Tétrahydro-1,4a,9b-triméthyl-1,4-méthanodibenzofuranne-8-yl)-2-oxoéthoxy]-2-hydroxy benzoate de benzyle
Acide 4-[2-(1,2,3,4-Tétrahydro-1,4a,9b-triméthyl-1,4-méthanodibenzofuranne-8-yl)-2-oxoéthoxy]-2-hydroxybenzoïque
Acide 4-[2-(1,2,3,4-Tétrahydro-1,4a,9b-triméthyl-1,4-méthanodibenzofuranne-8-yl)-2-hydroxyéthoxy]-2-hydroxybenzoïque
Acide 4-[2-(1,2,3,4-Tétrahydro-1,4a,9b-triméthyl-1,4-méthanodibenzofuranne-8-yl)-éthoxy]-2-hydroxybenzoïque
4-[(1,2,3,4-Tétrahydro-1,4a,9b-triméthyl-1,4-méthanodibenzofuranne-8-yl)-carbonyl] cinnamate d'éthyle
Acide 4-[(1,2,3,4-Tétrahydro-1,4a,9b-triméthyl-1,4-méthanodibenzofuranne-8-yl)-carbonyl] cinnamique
2-[3-(1 ,2,3,4-Tétrahydro-1 ,4a,9b-triméthyl-1,4-méthanodibenzofuranne-8-yl)-3-oxo-1-(E)-propényl]-4 thiophènecarboxylate d'éthyle
Acide 2-[3-(1,2,3,4-Tétrahydro-1,4a,9b-triméthyl-1,4-méthanodibenzofuranne-8-yl)-3-oxo-1-(E)-propényl]-4 thiophènecarboxylique
Acide-4-[3-(1,2,3,4-Tetrahydro-1,4a,9b-triméthyl-1,4-methanodibenzofuranne-8-yl)-3-oxo-(E)-1-propényl]-benzoïque.

13. Médicament, caractérisé en ce qu'il consiste en l'un des composés définis selon l'une quelconque des revendications 1 à 12.

14. Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 12.

15. Composition selon la revendication 14, caractérisée par le fait qu'elle contient de 0,001 à environ 5 % en poids d'un composé de formule (I).

16. Utilisation d'un composé selon l'une quelconque des revendications 1 à 12 pour la préparation d'une composition pharmaceutique destinée au traitement des affections dermatologiques, rhumatismales, respiratoires ainsi qu'ophtalmologiques.

17. Composition cosmétique pour l'hygiène corporelle et capillaire, caractérisée par le fait qu'elle contient, dans un véhicule cosmétique approprié, au moins un composé de formule (I) selon l'une quelconque des revendications 1 à 12.

18. Composition cosmétique selon la revendication 17, caractérisée par le fait qu'elle contient le composé de formule (I) à une concentration comprise entre 0,001 et 3 % en poids.

## Claims

1. Compounds, characterized in that they possess the following formula (I): 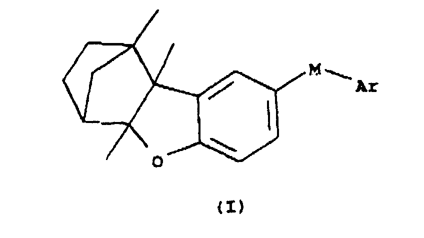 in which:
* Ar represents one of the radicals of the following formulae (II)-(VI): 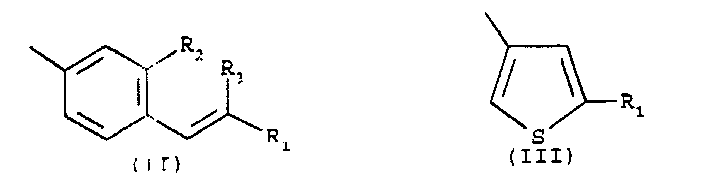 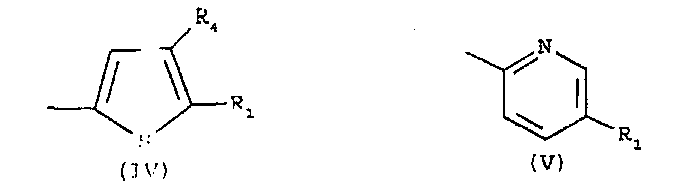 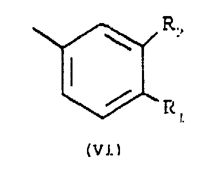
* M represents a bivalent radical chosen from the group consisting of the radicals of the following formulae (it being possible for the formulae given to be read from left to right or vice versa) :
(a) -CR₅R₆- 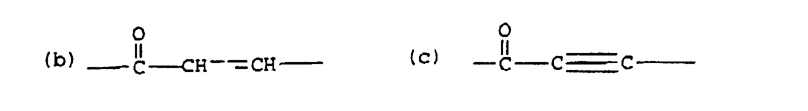 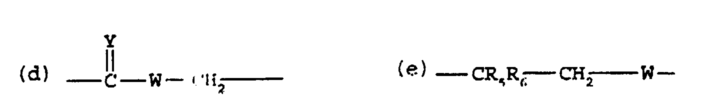
on the understanding that, in all the foregoing:
- when M represents a radical of formula (a), Ar does not represent a radical of formula (VI),
- R₁ represents:
(i) a hydrogen atom,
(ii) a -CH₃ radical,
(iii) a radical (CH₂)ₘ-O-R₈,
(iv) a radical -OR₈,
(v) a radical 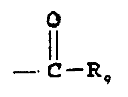
(vi) a radical -S(O)ₜR₉,
m, t, R₈ and R₉ having the meanings given below,
- R₂ represents a hydrogen atom or a radical -OR₈,
R₈ having the meaning given below,
- R₃ represents a hydrogen atom or an alkyl radical having from 1 to 6 carbon atoms,
- R₄ has the same meaning as R₁, on condition that at least one of the 2 radicals R₁ and R₄ is a hydrogen atom,
- either R₅ and R₆ independently represent a hydrogen atom, an alkyl radical having from 1 to 6 carbon atoms or a radical -(X)ₙ-(CH₂)ₚ-R₇, or R₅ and R₆ taken together can form an oxo (=O) group, a thicetone (=S) or oxime group, a group (R₁₁-O-N=), an epoxy or cyclopropyl group, a cycloalkyl group optionally substituted with a halogen atom or an alkyl radical having from 1 to 6 carbon atoms, or a dioxolane (-O-(CH₂)_{q}-O-) group with q equal to 2 or 3,
X, n, p, R₇ and R₁₁ having the meanings given below,
- R₇ representing a hydrogen atom or a radical -(CO)ᵣ-R₁₀ r and R₁₀ having the meanings given below,
- R₈ represents a hydrogen atom, an alkyl radical having from 1 to 6 carbon atoms or an acyl radical having from 1 to 6 carbon atoms,
- R₉ represents:
(i) a hydrogen atom,
(ii) a radical -N(R'R"),
(iii) a radical -OR₁₁,
R', R" and R₁₁ having the meanings given below,
- R₁₀ represents:
- a hydrogen atom,
- an alkyl radical,
- an alkenyl radical,
- an alkynyl radical,
- an aryl radical,
- a radical -OR₁₁,
- a radical -N(R'R"),
R', R" and R₁₁ having the meanings given below,
- R₁₁ represents a hydrogen atom, an alkyl radical having from 1 to 20 carbon atoms, a mono- or polyhydroxyalkyl radical, an aryl radical optionally substituted with one or more halogen atoms, a hydroxyl or nitro function or a methoxy group; an aralkyl radical optionally substituted with one or more halogen atoms, a hydroxyl or nitro function or a methoxy group; or a sugar residue,
- R' and R", which may be identical or different, represent a hydrogen atom, an alkyl radical having from 1 to 6 carbon atoms, a mono- or polyhydroxyalkyl radical, an aryl radical optionally substituted with one or more halogen atoms, a hydroxyl or nitro function or a methoxy group; an aralkyl radical optionally substituted with one or more halogen atoms, a hydroxyl or nitro function or a methoxy group; an amino acid or peptide or sugar residue, or alternatively R' and R" taken together form a heterocycle,
- W represents an oxygen or sulphur atom or a group -NR₁₂, R₁₂ having the meaning given below,
- R₁₂ represents a hydrogen atom or a -CH₃ radical,
- X and Y independently represent an oxygen atom or a sulphur atom,
- m and p, which are integers, vary independently from 0 to 10, on condition that:
when R₇ represents a radical -(CO)ᵣ-R₁₀ and R₁₀ represents a radical -OR₁₁, p cannot take the value 0,
- n and r can independently have the value 0 or 1,
- t is equal to 0, 1 or 2,
and the optical and geometrical isomers of the said compounds of formula (I), as well as their salts in the case where R₁, R₄ or R₇ represents an acid function.

2. Compounds according to Claim 1, characterized in that they take the form of salts by the addition of a base or acid.

3. Compounds according to one of the preceding claims, characterized in that the alkyl radical having from 1 to 6 carbon atoms is chosen from the group consisting of methyl, ethyl, isopropyl, butyl, tert-butyl and hexyl radicals.

4. Compounds according to one of the preceding claims, characterized in that the acyl radical having from 1 to 6 carbon atoms is chosen from the group consisting of acetyl, propionyl and pivaloyl radicals.

5. Compounds according to one of the preceding claims, characterized in that the monohydroxyalkyl radical is chosen from the group consisting of 2-hydroxyethyl, 2-hydroxypropyl and 3-hydroxypropyl radicals.

6. Compounds according to one of the preceding claims, characterized in that the polyhydroxyalkyl radical is chosen from the group consisting of 2,3-dihydroxypropyl, 2,3,4-trihydroxybutyl and 2,3,4,5-tetrahydroxypentyl radicals and the pentaerythritol residue.

7. Compounds according to one of the preceding claims, characterized in that the aryl radical is a phenyl radical optionally substituted with one or more halogen atoms, a hydroxyl or nitro function or a methoxy group.

8. Compounds according to one of the preceding claims, characterized in that the aralkyl radical is a benzyl or phenethyl radical optionally substituted with one or more halogen atoms, a hydroxyl or nitro function or a methoxy group.

9. Compounds according to one of the preceding claims, characterized in that the sugar residue is a residue derived from glucose, from galactose, from mannose or from glucuronic acid.

10. Compounds according to one of the preceding claims, characterized in that the heterocycle is a piperidino, morpholino, pyrrolidino or piperazino radical optionally substituted at position 4 with a C₁-C₆ alkyl radical or a mono- or polyhydroxyalkyl radical.

11. Compounds according to any one of the preceding claims, characterized in that they possess the following formula (Ia):

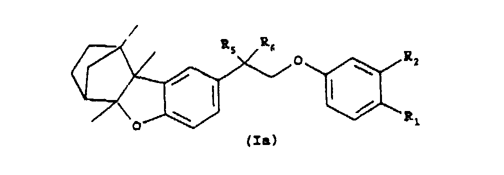

12. Compounds according to any one of Claims 1 to 10, characterized in that they are chosen from the group consisting of:
Methyl 4-[(E)-3-(1,2,3,4-tetrahydro-1,4a,9b-trimethyl-1,4-methanodibenzofuran-8-yl)-3-oxo-1-propenyl]benzoate
4-[(E)-3-11,2,3,4-Tetrahydro-1,4a,9b-trimethyl-1,4-methanodibenzofuran-8-yl)-3-oxo-1-propenyl]benzoic acid
Methyl 4-[(E)-3-(1,2,3,4-tetrahydro-1,4a,9b-trimethyl-1,4-methanodibenzofuran-8-yl)-3-oxo-1-propynyl]benzoate
4-[(E)-3-(1,2,3,4-Tetrahydro-1,4a,9b-trimethyl-1,4-methanodibenzofuran-8-yl)-3-oxo-1-propynyl]benzoic acid
Benzyl 4-[2-(1,2,3,4-tetrahydro-1,4a,9b-trimethyl-1,4-methanodibenzofuran-8-yl)-2-oxoethoxy]benzoate
4-[2-(1,2,3,4-Tetrahydro-1,4a,9b-trimethyl-1,4-methanodibenzofuran-8-yl)-2-oxoethoxy]benzoic acid
4-[2-(1,2,3,4-Tetrahydro-1,4a,9b-trimethyl-1,4-methanodibenzofuran-8-yl)-2-hydroxyethoxy]benzoic acid
Benzyl 4-[2-(1,2,3,4-tetrahydro-1,4a,9b-trimethyl-1,4-methanodibenzofuran-8-yl)-2-oxoethoxyl-2-hydroxybenzoate
4-[2-(1,2,3,4-Tetrahydro-1,4a,9b-trimethyl-1,4-methanodibenzofuran-8-yl)-2-oxoethoxy]-2-hydroxybenzoic acid
4-[2-(1,2,3,4-Tetrahydro-1,4a,9b-trimethyl-1,4-methanodibenzofuran-8-yl)-2-hydroxyethoxy)-2-hydroxybenzoic acid
4-[2-(1,2,3,4-Tetrahydro-1,4a,9b-trimethyl-1,4-methanodibenzofuran-8-yl)ethoxy]-2-hydroxybenzoic acid
Ethyl 4-[(1,2,3,4-tetrahydro-1,4a,9b-trimethyl-1,4-methanodibenzofuran-8-yl)carbonyl]cinnamate
4-[(1,2,3,4-Tetrahydro-1,4a,9b-trimethyl-1,4-methanodibenzofuran-8-yl)carbonyl]cinnamic acid
Ethyl 2-[(E)-3-(1,2,3,4-tetrahydro-1,4a,9b-trimethyl-1,4-methanodibenzofuran-8-yl)-3-oxo-1-propenyl]-4-thiophenecarboxylate
2-[(E)-3-(1,2,3,4-Tetrahydro-1,4a,9b-trimethyl-1,4-methanodibenzofuran-8-yl)-3-oxo-1-propenyl]-4-thiophenecarboxylic acid
4-[(E)-3-(1,2,3,4-Tetrahydro-1,4a,9b-trimethyl-1,4-methanodibenzofuran-8-yl)-3-oxo-1-propenyl]benzoic acid.

13. Medicinal product, characterized in that it consists of one of the compounds defined according to any one of Claims 1 to 12.

14. Pharmaceutical composition, characterized in that it contains, in a vehicle suitable for administration via the enteral, parenteral, topical or ocular route, at least one compound of formula (I) according to any one of Claims 1 to 12.

15. Composition according to Claim 14, characterized in that it contains from 0.001 to approximately 5 % by weight of a compound of formula (I).

16. Use of a compound according to any one of Claims 1 to 12 for the preparation of a pharmaceutical composition intended for the treatment of dermatological, rheumatic, respiratory and also ophthalmological complaints.

17. Cosmetic composition for body and hair hygiene, characterized in that it contains, in a suitable cosmetic vehicle, at least one compound of formula (I) according to any one of Claims 1 to 12.

18. Cosmetic composition according to Claim 17, characterized in that it contains the compound of formula (I) at a concentration of between 0.001 and 3 % by weight.

## Patentansprüche

1. Verbindungen, dadurch gekennzeichnet, daß sie der folgenden Formel (I) entsprechen: 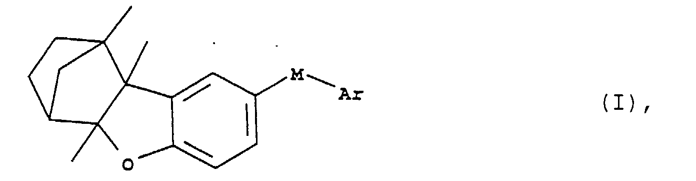 worin bedeuten:
* Ar eine Gruppe der folgenden Formeln (II) bis (VI): 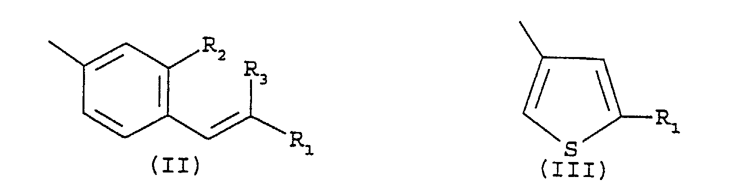 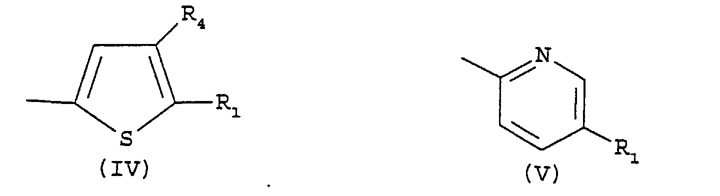 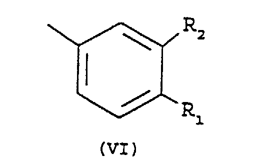
* M eine zweiwertige Gruppe, die unter den Gruppen der folgenden Formeln ausgewählt ist (wobei die angegebenen Formeln von links nach rechts und umgekehrt gelesen werden können) :
(a) -CR₅R₆-, 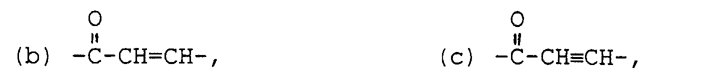 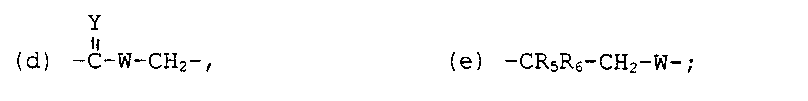
mit der Maßgabe, daß
- Ar keine Gruppe der Formel (VI) bedeutet, wenn M die Gruppe der Formel (a) ist,
- R₁ bedeutet:
(i) Wasserstoff,
(ii) die Gruppe -CH₃,
(iii) eine Gruppe -(CH₂)ₘ-O-R₈,
(ii) eine Gruppe -O-R₈,
(v) eine Gruppe 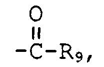
(vi) eine Gruppe -S(O)ₜ-R₉,
wobei m, t, R₈ und R₉ die nachstehend angegebenen Bedeutungen aufweisen,
- R₂ ein Wasserstoffatom oder eine Gruppe -OR₈ bedeutet, wobei R₈ die nachstehend angegebenen Bedeutungen aufweist,
- R₃ ein Wasserstoffatom oder eine Alkylgruppe mit 1 bis 6 Kohlenstoffatomen bedeutet,
- R₄ die Bedeutungen von R₁ aufweist, mit der Maßgabe, daß mindestens eine der beiden Gruppen R₁ oder R₄ Wasserstoff bedeutet,
- R₅ und R₆ entweder unabhängig voneinander ein Wasserstoffatom, eine Alkylgruppe mit 1 bis 6 Kohlenstoffatomen oder eine Gruppe -(X)ₙ-(CH₂)ₚ-R₇ bedeuten, oder R₅ und R₆ gemeinsam eine Oxogruppe (=O), Thioketon-gruppe (=S), eine Oximgruppe oder eine Gruppe (R₁₁-O-N=), eine Epoxygruppe, eine Cyclopropylgruppe, eine Cycloalkylgruppe, die gegebenenfalls mit einem Halogenatom oder einer Alkylgruppe mit 1 bis 6 Kohlenstoffatomen substituiert ist, oder auch eine Dioxolangruppe (-O-(CH₂)_{q}-O-) bilden können, worin q 2 oder 3 bedeutet, wobei X, n, p, R₇ und R₁₁ die nachfolgend angegebenen Bedeutungen aufweisen,
- R₇ ein Wasserstoffatom oder eine Gruppe -(CO)ᵣ-R₁₀ bedeutet, wobei r und R₁₀ die nachfolgend angegebenen Bedeutungen aufweisen,
- R₈ ein Wasserstoffatom, eine Alkylgruppe mit 1 bis 6 Kohlenstoffatomen oder eine Acylgruppe mit 1 bis 6 Kohlenstoffatomen bedeutet,
- R₉ bedeutet:
(i) ein Wasserstoffatom,
(ii) eine Gruppe -N(R',R"),
(iii) eine Gruppe -OR₁₁,
worin R', R" und R₁₁ die nachfolgend angegebenen Bedeutungen aufweisen,
- R₁₀ bedeutet:
- ein Wasserstoffatom,
- eine Alkylgruppe,
- eine Alkenylgruppe,
- eine Alkinylgruppe,
- eine Arylgruppe,
- eine Gruppe -OR₁₁,
- eine Gruppe -N(R',R"),
worin R', R" und R₁₁ die nachfolgend angegebenen Bedeutungen aufweisen,
- R₁₁ ein Wasserstoffatom, eine Alkylgruppe mit 1 bis 20 Kohlenstoffatomen, eine Mono- oder Polyhydroxyalkylgruppe, eine gegebenenfalls mit einem oder mehreren Halogenatomen, einer Hydroxygruppe, einer Nitrogruppe oder einer Methoxygruppe substituierte Arylgruppe, eine gegebenenfalls mit einem oder mehreren Halogenatomen, einer Hydroxygruppe, einer Nitrogruppe oder einer Methoxygruppe substituierte Aralkylgruppe oder einen Zuckerrest bedeutet,
- R' und R", die identisch oder voneinander verschieden sind, bedeuten: Wasserstoff, eine Alkylgruppe mit 1 bis 6 Kohlenstoffatomen, eine Mono- oder Polyhydroxyalkylgruppe, eine Arylgruppe, die gegebenenfalls mit einem oder mehreren Halogenatomen, einer Hydroxygruppe, einer Nitrogruppe oder einer Methoxygruppe substituiert ist, eine Aralkylgruppe, die gegebenenfalls mit einem oder mehreren Halogenatomen, einer Hydroxygruppe, einer Nitrogruppe oder einer Methoxygruppe substituiert ist, oder einen Aminosäure-, Petid- oder Zuckerrest oder R' und R" bilden gemeinsam einen Heterocyclus,
- W ein Sauerstoff- oder Schwefelatom oder eine Gruppe -NR₁₂ bedeutet, wobei R₁₂ die nachfolgend angegebene Bedeutung aufweist,
- R₁₂ ein Wasserstoffatom oder die Gruppe -CH₃ bedeutet,
- X und Y unabhängig voneinander ein Sauerstoff- oder ein Schwefelatom bedeuten,
- m und p ganze Zahlen sind, die unabhängig voneinander im Bereich von 0 bis 10 liegen, mit der Maßgabe, daß p nicht den Wert 0 annehmen kann, wenn R₇ eine Gruppe -(CO)ᵣ-R₁₀ und R₁₀ eine Gruppe -OR₁₁ bedeutet,
- n und r unabhängig voneinander 0 oder 1 bedeuten, und
- t 0, 1 oder 2 ist;
und die optischen und geometrischen Isomere der Verbindungen der Formel (I) sowie die Salze dieser Verbindungen, wenn R₁, R₄ oder R₇ eine Säuregruppe bedeuten.

2. Verbindungen nach Anspruch 1, dadurch gekennzeichnet, daß sie durch Zugabe einer Säure oder einer Base in Salzform vorliegen.

3. Verbindungen nach einem der vorhergehenden Ansprüche, dadurch gekennzeichnet, daß die Alkylgruppe mit 1 bis 6 Kohlenstoffatomen unter Methyl, Ethyl, Isopropyl, Butyl, tert.-Butyl und Hexyl ausgewählt ist.

4. Verbindungen nach einem der vorhergehenden Ansprüche, dadurch gekennzeichnet, daß die Acylgruppe mit 1 bis 6 Kohlenstoffatomen unter Acetyl, Propionyl und Pivaloyl ausgewählt ist.

5. Verbindungen nach einem der vorhergehenden Ansprüche, dadurch gekennzeichnet, daß die Monohydroxyalkylgruppe unter 2-Hydroxyethyl, 2-Hydroxypropyl oder 3-Hydroxypropyl ausgewählt ist.

6. Verbindungen nach einem der vorhergehenden Ansprüche, dadurch gekennzeichnet, daß die Polyhydroxyalkylgruppe unter 2,3-Dihydroxypropyl, 2,3,4-Trihydroxybutyl, 2,3,4,5-Tetrahydroxypentyl oder der Pentaerythritgruppe ausgewählt ist.

7. Verbindungen nach einem der vorhergehenden Ansprüche, dadurch gekennzeichnet, daß die Arylgruppe eine Phenylgruppe ist, die gegebenenfalls mit einem oder mehreren Halogenatomen, einer Hydroxygruppe, einer Nitrogruppe oder einer Methoxygruppe substituiert ist.

8. Verbindungen nach einem der vorhergehenden Ansprüche, dadurch gekennzeichnet, daß die Aralkylgruppe unter den Benzyl- oder Phenethylgruppen ausgewählt ist, die gegebenenfalls mit einem oder mehreren Halogenatomen, einer Hydroxygruppe, einer Nitrogruppe oder einer Methoxygruppe substituiert sind.

9. Verbindungen nach einem der vorhergehenden Ansprüche, dadurch gekennzeichnet, daß der Zuckerrest unter den Resten von Glucose, Galactose, Mannose oder Glucuronsäure ausgewählt ist.

10. Verbindungen nach einem der vorhergehenden Ansprüche, dadurch gekennzeichnet, daß die heterocyclischen Gruppen unter den Gruppen Piperidino, Morpholino, Pyrrolidino oder Piperazino ausgewählt sind, die gegebenenfalls in 4-Stellung mit einer C₁₋₆-Alkylgruppe oder Mono- oder Polyhydroxyalkylgruppe substituiert sind.

11. Verbindungen nach einem der vorhergehenden Ansprüche, dadurch gekennzeichnet, daß sie der folgenden Formel (Ia) entsprechen:

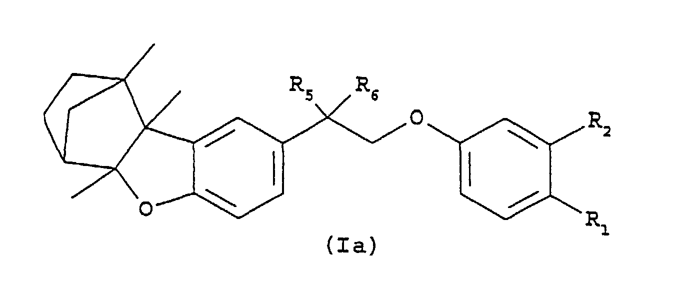

12. Verbindungen nach einem der Ansprüche 1 bis 10, dadurch gekennzeichnet, daß sie ausgewählt sind unter:
Methyl-4-[3-(1,2,3,4-tetrahydro-1,4a,9b-trimethyl-1,4-methano-dibenzofuran-8-yl)-3-oxo-1-(E)-propenyl]-benzoat,
4-[3-(1,2,3,4-Tetrahydro-1,4a,9b-trimethyl-1,4-methano-dibenzofuran-8-yl)-3-oxo-1-(E)-propenyl]benzoesäure,
Methyl-4-[3-(1,2,3,4-tetrahydro-1,4a,9b-trimethyl-1,4-methano-dibenzofuran-8-yl)-3-oxo-1-(E)-propinyl]-benzoat,
4-[3-(1,2,3,4-Tetrahydro-1,4a,9b-trimethyl-1,4-methano-dibenzofuran-8-yl)-3-oxo-1-(E)-propinyl]-benzoesäure,
Benzyl-4-[2-(1,2,3,4-tetrahydro-1,4a,9b-trimethyl-1,4-methano-dibenzofuran-8-yl)-2-oxoethoxy]-benzoat,
4-[2-(1,2,3,4-Tetrahydro-1,4a,9b-trimethyl-1,4-methano-dibenzofuran-8-yl)-2-oxoethoxy]-benzoesäure,
4-[2-(1,2,3,4-Tetrahydro-1,4a,9b-trimethyl-1,4-methano-dibenzofuran-8-yl)-2-hydroxyethoxy]-benzoesäure,
Benzyl-4-[2-(1,2,3,4-tetrahydro-1,4a,9b-trimethyl-1,4-methano-dibenzofuran-8-yl)-2-oxoethoxy]-2-hydroxybenzoat,
4-[2-(1,2,3,4-Tetrahydro-1,4a,9b-trimethyl-1,4-methano-dibenzofuran-8-yl)-2-oxoethoxy]-2-hydroxybenzoesäure,
4-[2-(1,2,3,4-Tetrahydro-1,4a,9b-trimethyl-1,4-methano-dibenzofuran-8-yl)-2-hydroxyethoxy]-2-hydroxybenzoesäure,
4-[2-(1,2,3,4-Tetrahydro-1,4a,9b-trimethyl-1,4-methano-dibenzofuran-8-yl)-ethoxy]-2-hydroxy-benzoesäure,
Ethyl-4-[(1,2,3,4-tetrahydro-1,4a,9b-trimethyl-1,4-methano-dibenzofuran-8-yl)-carbonyl]-cinnamat,
4-[(1,2,3,4-Tetrahydro-1,4a,9b-trimethyl-1,4-methano-dibenzofuran-8-yl)-carbonyl]-zimtsäure,
Ethyl-2-[3-(1,2,3,4-tetrahydro-1,4a,9b-trimethyl-1,4-methano-dibenzofuran-8-yl)-3-oxo-1-(E)-propenyl]-4-thiophencarboxylat,
2-[3-(1,2,3,4-Tetrahydro-1,4a,9b-trimethyl-1,4-methano-dibenzofuran-8-yl)-3-oxo-1-(E)-propenyl]-4-thiophencarbonsäure, und
4-[3-(1,2,3,4-Tetrahydro-1,4a,9b-trimethyl-1,4-methano-dibenzofuran-8-yl)-3-oxo-1-(E)-propenyl]-benzoesäure.

13. Arzneimittel, dadurch gekennzeichnet, daß es aus einer Verbindung nach einem der Ansprüche 1 bis 12 besteht.

14. Pharmazeutische Zusammensetzung, dadurch gekennzeichnet, daß sie in einem für eine enterale, parenterale, topische oder okulare Verabreichung geeigneten Träger mindestens eine Verbindung der Formel (I) nach einem der Ansprüche 1 bis 12 enthält.

15. Zusammensetzung nach Anspruch 14, dadurch gekennzeichnet, daß sie 0,001 bis etwa 5 Gew.-% einer Verbindung der Formel (I) enthält.

16. Verwendung einer Verbindung nach einem der Ansprüche 1 bis 12 zur Herstellung einer pharmazeutischen Zusammensetzung, die zur Behandlung von dermatologischen Erkrankungen, rheumatischen Erkrankungen, Erkrankungen der Atemwege und ophthalmologischen Erkrankungen bestimmt ist.

17. Kosmetische Zusammensetzung zur Körper- und Haarhygiene, dadurch gekennzeichnet, daß sie in einem kosmetisch akzeptablen Träger mindestens eine Verbindung der Formel (I) nach einem der Ansprüche 1 bis 12 enthält.

18. Kosmetische Zusammensetzung nach Anspruch 17, dadurch gekennzeichnet, daß sie die Verbindung der Formel (I) in einer Konzentration im Bereich von 0,001 bis 3 Gew.-% enthält.
